(19) Europäisches Patentamt
European Patent Office
Office européen des brevets

(11) **EP 2 936 145 B1**

(12) **FASCICULE DE BREVET EUROPEEN**

(45) Date de publication et mention
de la délivrance du brevet:
**13.06.2018 Bulletin 2018/24**

(21) Numéro de dépôt: **13818740.6**

(22) Date de dépôt: **19.12.2013**

(51) Int Cl.:
***G01N 33/00*** (2006.01)  ***G01N 1/22*** (2006.01)

(86) Numéro de dépôt international:
**PCT/EP2013/077538**

(87) Numéro de publication internationale:
**WO 2014/096287 (26.06.2014 Gazette 2014/26)**

(54) **DISPOSITIF ET PROCEDE DE DISCRIMINATION D'UN GAZ DANS UN ECHANTILLON**

VORRICHTUNG UND VERFAHREN ZUR UNTERSCHEIDUNG VON GASEN IN EINER PROBE

DEVICE AND METHOD FOR DISCRIMINATING A GAS IN A SAMPLE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.12.2012 FR 1262680**

(43) Date de publication de la demande:
**28.10.2015 Bulletin 2015/44**

(60) Demande divisionnaire:
**18170505.4**

(73) Titulaire: **Aneolia
91480 Varennes-Jarcy (FR)**

(72) Inventeurs:
• **GOSSE, Thierry
F-91140 Villebon-sur-Yvette (FR)**
• **LACARRERE, Philippe
F-77370 Nangis (FR)**
• **SCHALLER, Eric
F-91480 Varrennes-Jarcy (FR)**

(74) Mandataire: **Pontet Allano & Associes
Parc Les Algorithmes, Bâtiment Platon
CS 70003 Saint-Aubin
91192 Gif-sur-Yvette Cedex (FR)**

(56) Documents cités:
**WO-A1-2008/046420   US-A1- 2011 303 019**

• **SEARS W M ET AL: "Surface adsorption and gas
consumption in restricted flow, thermally driven,
gas sensors", SEMICONDUCTOR SCIENCE AND
TECHNOLOGY, IOP PUBLISHING LTD, GB, vol.
5, no. 1, 1 janvier 1990 (1990-01-01) , pages 45-53,
XP020032103, ISSN: 0268-1242, DOI:
10.1088/0268-1242/5/1/005**

## Description

### Domaine technique

La présente invention concerne un dispositif de test d'un échantillon. Elle concerne aussi un procédé mis en oeuvre par ce dispositif.

### Etat de la technique antérieure

On connaît des systèmes de test d'échantillons, par exemple pour mesurer le taux d'un gaz donné à l'intérieur de l'échantillon ou pour mesurer une fuite ou un problème d'étanchéité de l'échantillon.

Un problème récurrent des solutions de l'état de l'art est qu'elles sont trop chères, trop longues (temps de réponse typique d'une douzaine de secondes pour une mesure par infrarouge de taux de $CO_2$), ou pas assez précises (taille minimum d'un trou de fuite mesurable de 5 $\mu$m avec une surpression relative de 500 mbar, ou avec un balayage d'Hélium dans l'enceinte à mesurer).

L'article « Surface adsorption and gas consumption in restricted flow, thermally driven, gas sensors » de Sears W. M. et al. paru dans « semiconductor science and technology » vol.5, 1 janvier 1990, pages 45-53, décrit un dispositif comprenant des capteurs de gaz de type Taguchi montés dans une petite chambre avec une bande de platine, et ayant accès à une atmosphère extérieure de manière restreinte via une ouverture étroite.

Le document WO 2008/046420 A1 décrit un procédé de détermination du volume d'un espace clos. Le procédé consiste à remplir ledit espace clos à l'aide d'un premier gaz et à rincer au moins partiellement ledit espace clos à l'aide d'un second gaz de composition connnue. Le rinçage comprend un certain nombre d'étapes de rinçage successives, au cours desquelles des quantités connues du second gaz sont injectées dans l'espace clos inconnu. La modification de concentration dans ledit espace clos inconnu d'un gaz de mesure présent dans le premier gaz ou présent dans le second gaz avant et après une telle étape de rinçage est mesurée. Le volume de l'espace clos inconnu est calculé, sur la base de la modification de concentration dudit gaz de mesure.

Le document US2011/303019 A1 décrit un appareil et un procédé de surveillance d'un flux de fluide multiphasique circulant dans un tuyau. Le procédé comprend les étapes consistant à : a) obtenir une valeur de pression d'écoulement et une valeur de température d'écoulement pour le flux de fluide multiphasique dans le tuyau ; b) détecter le flux de fluide au moyen d'un débitmètre de fluide pouvant se fixer à l'extérieur du tuyau, le débitmètre comprenant un réseau spatial d'au moins deux capteurs disposés à des positions axiales différentes le long du tuyau et générant des signaux de vitesse d'écoulement indiquant une vitesse du flux de fluide circulant dans le tuyau ; c) injecter sélectivement au moins un traceur dans le flux de fluide qui passe dans le tuyau à un débit et une concentration d'injection connus ; d) détecter le traceur dans un échantillon du flux de fluide et générer des signaux de concentration du traceur indiquant la concentration du traceur dans le flux de fluide ; et e) déterminer un ou plusieurs parmi un débit de composant gazeux, un débit de composant huileux et un débit de composant aqueux en utilisant un ou plusieurs éléments parmi la valeur de pression d'écoulement, la valeur de température d'écoulement, les signaux de vitesse d'écoulement et les signaux de concentration du traceur.

Le but de la présente invention est de proposer un dispositif et un procédé de test d'un échantillon ayant au moins un des avantages techniques parmi les suivants :

- faible coût de production par rapport à l'état de l'art,
- grande rapidité d'une mesure par rapport à l'état de l'art, et
- grande résolution de mesure par rapport à l'état de l'art.

### Exposé de l'invention

Cet objectif est atteint avec un dispositif et un procédé de test d'un échantillon par flux gazeux selon les revendications 1 et 18, respectivement.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :

- la figure 1 est une vue schématique de coupe de profil d'un dispositif selon l'invention, qui est le mode de réalisation préféré de l'invention, et sur laquelle est illustré un flux de gaz lorsque ce dispositif est dans une position d'analyse de gaz par aspiration ou dans une position de calibration,
- la figure 2 illustre schématiquement le circuit pneumatique du dispositif de la figure 1 dans la position d'analyse de gaz par aspiration ou dans la position de calibration,
- la figure 3 est une vue schématique de coupe de dessous d'une partie du dispositif de la figure 1, et sur laquelle est illustré un flux de gaz lorsque ce dispositif est dans la position d'analyse de gaz par aspiration ou dans la position de calibration,
- la figure 4 illustre schématiquement le circuit pneumatique du dispositif de la figure 1 dans une position de dilution ou dans une autre position de calibration,
- la figure 5 est une vue schématique de coupe de profil du dispositif de la figure 1, et sur laquelle est illustré un flux de gaz lorsque ce dispositif est dans une position de détection de fuite par expiration, ce mode de réalisation étant en dehors de la portée de la présente invention.

- la figure 6 illustre schématiquement le circuit pneumatique du dispositif de la figure 1 dans la position de détection de fuite par expiration, ce mode de réalisation étant en dehors de la portée de la présente invention
- la figure 7 illustre schématiquement le circuit pneumatique du dispositif de la figure 1 dans une position de gonflage rapide par expiration, ce mode de réalisation étant en dehors de la portée de la présente invention
- la figure 8 illustre schématiquement le circuit pneumatique du dispositif de la figure 1 dans une position d'éclatement par expiration, ce mode de réalisation étant en dehors de la portée de la présente invention

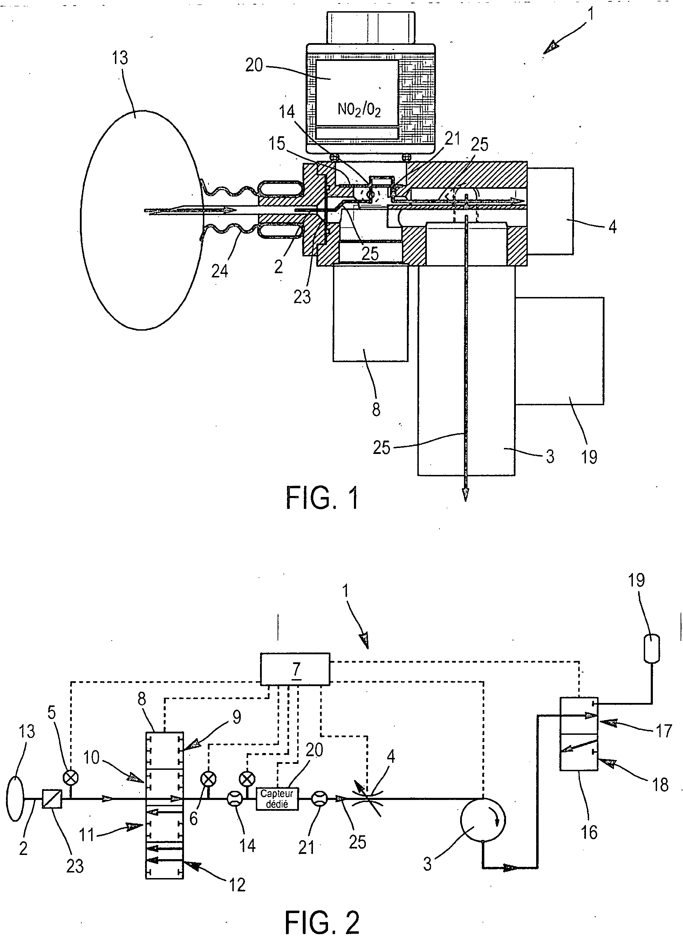

On va tout d'abord décrire, en référence aux figures 1 à 8, un mode de réalisation préféré de dispositif 1 selon l'invention. Le dispositif 1 est un sous-ensemble technique compact pouvant s'installer dans un système portatif ou s'intégrer au sein d'une installation fixe.

Le dispositif 1 est un dispositif de test d'un échantillon par flux gazeux.

Le dispositif 1 comprend un orifice 2. Cet orifice 2 est l'orifice d'entrée du creux d'une aiguille creuse, disposée au centre d'une ventouse 24 étanche agencée pour être plaquée contre un échantillon 13 (tel un sachet de produit alimentaire ou tout contenant présentant au moins une surface souple de dimension compatible pouvant être traversée par une aiguille). La ventouse évite le recours à des septums d'étanchéité pour effectuer un test sans pollution de l'air extérieur au contenant.

Le dispositif 1 comprend en outre des moyens 3 pour générer un flux de gaz 25 (gaz à analyser, gaz de dilution, gaz de fuite, gaz de calibration) dans le dispositif 1 le long d'au moins un chemin d'écoulement passant par l'orifice 2, par un débitmètre massique 4, et par une vanne 8 appelée vanne de sélection.

La vanne 8 est une vanne à plus de deux voies (d'entrée ou de sortie), ayant plusieurs positions possibles. Chaque position de la vanne 8 correspond à une configuration spécifique d'ouverture pour le passage du flux gazeux 25 ou de fermeture pour empêcher un tel passage entre certaines des voies d'entrée et sortie de la vanne 8.

La vanne 8 est de préférence une vanne proportionnelle (de préférence à tiroir).

La vanne 8 est par exemple une vanne réalisée à partir d'un électroaimant de marque Mécalectro, ou encore d'une vanne Parker.

L'orifice 2 et la vanne 8 sont communs à tous les chemins d'écoulement. Le long de cette partie commune des chemins d'écoulement est de préférence situé un élément micro-poreux de filtration 23.

Le filtre 23 est par exemple un filtre en PTFE de chez Millipore ou Sartorius.

Les moyens de génération 3 comprennent une turbine, ou plus généralement un générateur de flux réversible à vitesse contrôlée afin d'être asservie en débit ou en pression, par exemple de marque Papst.

Les moyens de génération 3 sont réversibles, c'est-à-dire qu'ils sont agencés pour générer aussi bien un flux gazeux 25 en aspiration qu'en expiration (i.e. dans un sens d'écoulement opposé à l'aspiration).

Une vanne 16 et l'orifice 2 délimite les deux extrémités de chacun des chemins d'écoulement.

La vanne 16 est une vanne à plus de deux voies (d'entrée ou de sortie), ayant plusieurs positions possibles. Selon la position de la vanne 16, la vanne 16 relie les moyens de génération 3 à l'atmosphère extérieure du dispositif 1 dans une première position 17 ou à une source 19 de gaz de référence dans une deuxième position 18. La vanne 16 est par exemple une vanne de marque Bosch ou bien Univer.

Le dispositif 1 comprend au moins un capteur de pression 5, 6, chaque capteur de pression 5, 6 étant agencé pour mesurer une pression $P_r$ du flux de gaz 25 le long d'au moins un des chemins d'écoulement. Plus exactement, la pression Pr mesurée par chaque capteur 5 ou 6 est une pression relative (respectivement dépression ou surpression) générée par le flux 25 (respectivement aspiré dans le dispositif 1 ou expiré du dispositif 1) par rapport à la pression absolue qui serait mesurée en l'absence de ce flux 25. Chaque capteur 5, 6 est par exemple un capteur piézorésistif de marque Honeywell, Freescale, ou Sensortechnics.

Le débitmètre massique 4 est agencé pour mesurer un paramètre représentatif du débit massique du flux de gaz le long de chaque chemin d'écoulement. Ce paramètre est typiquement une intensité électrique ou une tension électrique, et est de préférence proportionnel au débit massique du flux de gaz 25 ou relié au débit massique du flux de gaz 25 par un calcul programmé et/ou mémorisé au sein de moyens de calcul 7 du dispositif. Tous les éléments sensoriels et d'asservissement 5, 8, 6, 20, 4, 3, 16 sont reliés aux moyens de calcul 7 par une liaison électrique et/ou de transfert de données ou de commande (liaisons représentés en pointillés sur la figure 2). Les moyens de calcul et de commande 7 ne sont représentés schématiquement que sur la figure 2 pour ne pas surcharger les autres figures.

Dans le présent document, le mot « chaque » sert à désigner toute unité (par exemple capteur ou chemin d'écoulement) prise individuellement dans un ensemble. Dans le cas où cet ensemble comprend au moins une unité (i.e. par exemple « au moins un capteur » ou « au moins un chemin d'écoulement »), il existe donc un cas limite où l'ensemble comprend une seule unité (i.e. par exemple un seul capteur ou un seul chemin d'écoulement) et où le mot « chaque » désigne cette seule unité.

Les moyens de calcul 7 ne comprennent que des moyens techniques électroniques et/ou logiciels (de préférence électroniques), et comprennent une unité centrale d'ordinateur, et/ou un processeur, et/ou un circuit analogique ou numérique dédié, et/ou du logiciel.

Le débitmètre massique 4 est un débitmètre massique à conductibilité thermique.

Typiquement, le débitmètre massique 4 comprend un élément chauffant (source de chaleur) et deux sondes de températures. L'élément chauffant se trouve entre les deux sondes de températures de sorte que l'élément chauffant et les deux sondes de températures soient tous les trois alignés le long du sens d'écoulement du flux de gaz 25 au niveau du débitmètre massique. Le débitmètre massique 4, en fonction de la variation de température ou de quantité de chaleur entre les deux sondes de température bordant la source de chaleur, est agencé pour en déterminer le paramètre représentatif du débit massique du flux de gaz 25 passant par le débitmètre 4 (c'est-à-dire une masse de gaz passant par le chemin d'écoulement par unité de temps).

L'avantage d'un débitmètre massique, en particulier à conductibilité thermique, et qu'il a un très rapide temps de réponse. Il va donc permettre d'accéder à un diamètre de trou de fuite 22 ou à une quantification de la présence d'un gaz d'intérêt avec une très grande rapidité de mesure (temps de réponse typique de 3 millisecondes).

L'au moins un chemin d'écoulement comprend :

- un chemin d'aspiration débutant par l'orifice 2 (i.e. le flux de gaz entre dans le chemin d'aspiration par l'orifice 2),
- un chemin d'expiration se terminant par l'orifice 2 (i.e. le flux de gaz sort du chemin d'expiration par l'orifice 2), et
- un chemin de dilution se terminant par l'orifice 2 (i.e. le flux de gaz sort du chemin de dilution par l'orifice 2).

Tous ces chemins d'écoulement sont permis dans le dispositif 1 selon la position de la vanne 8 et le sens du flux 25 généré par les moyens de génération 3. La position de la vanne 8 et le sens du flux 25 généré par les moyens de génération 3 (expiration ou aspiration) à un instant donné détermine l'unique (zéro ou un parmi le chemin d'aspiration, le chemin d'expiration ou le chemin de dilution) chemin d'écoulement à travers lequel s'écoule le flux 25 de gaz à cet instant dans le dispositif 1.

Tous les chemins d'écoulement sont fermés

Pour une première position 9 de la vanne 8, la vanne 8 est fermée et le flux de gaz 25 généré par les moyens 3 ne peut s'écouler le long d'aucun chemin d'écoulement tel que défini précédemment.

Chemin d'aspiration

En référence aux figures 1 à 3, pour une deuxième position 10 de la vanne 8, et pour les moyens de génération 3 aspirant le flux de gaz 25, les moyens 3 pour générer le flux de gaz 25 sont agencés pour aspirer un gaz à analyser en provenance d'un échantillon 13 de sorte que ce gaz à analyser s'écoule dans le dispositif 1 le long du chemin d'aspiration.

Le gaz à analyser comprend par exemple :

- 0 à 100% d'un gaz de mélange comprenant une ou plusieurs molécules (par exemple $N_2$ et $O_2$), chacune de ces molécules ayant avec les autres molécules du gaz de mélange un écart de conductibilité thermique d'au plus 10% (de préférence d'au plus 5%) pour des conditions identiques de température et de pression (typiquement, pour chaque paire de deux molécules du gaz de mélange ayant une conductibilité thermique respectivement $D_i$ et $D_j$ pour des conditions identiques de température (température du flux 25 lors de la mesure de pression $P_r$, typiquement 20°C) et de pression (Pression de mesure $P_r$),

$$\frac{D_i - D_j}{D_i} \leq 10\% \qquad \frac{D_i - D_j}{Dj} \leq 10\%,$$

on a et voir

$$\frac{D_i - D_j}{D_i} \leq 5\%$$

de préférence et

$$\frac{D_i - D_j}{Dj} \leq 5\% \ );$$

ce seuil qui est de manière optimale fixé à 5 ou 10 % peut être aussi supérieur à 10% (20%, 30%, etc...) dans d'autres modes de réalisation, mais plus ce seuil va être élevé, moins bonne va être la résolution du dispositif selon l'invention ; et

- 0 à 100% d'un gaz d'intérêt comprenant uniquement une ou plusieurs molécules (par exemple $NO_2$ et/ou $CO_2$) ayant entre elles un écart de conductibilité thermique inférieur ou égal à 10% (de préférence inférieur ou égal à 5%) pour des conditions identiques de température et de pression (typiquement, pour chaque paire de deux molécules du gaz d'intérêt ayant une conductibilité thermique respectivement $C_i$ et $C_j$ pour des conditions identiques de température (température du flux 25 lors de la mesure de pression $P_r$, typiquement 20°C) et de pression (Pression de mesure $P_r$), on a

$$\frac{C_i - C_j}{C_i} \leq 10\%$$

sion de mesure $P_r$), on a et

$$\frac{C_i - C_j}{Cj} \leq 10\%,$$

voir de préférence

$$\frac{C_i - C_j}{C_i} \leq 5\% \qquad \frac{C_i - C_j}{Cj} \leq 5\% ).$$

et Ce seuil qui est de manière optimale fixé à 5 ou 10 % peut être aussi supérieur à 10% (20%, 30%, etc...) dans d'autres modes de réalisation, mais plus ce seuil va être élevé, moins bonne va être la résolution du dispositif selon l'invention. Chaque molécule du gaz d'intérêt a une conductibilité thermique différente de la conductibilité thermique de chacune des molécules du gaz de mélange d'au moins 20%, de préférence d'au moins 30%, pour des conditions identiques de température et de pression (typiquement, pour chaque molécule du gaz d'intérêt ayant une conductibilité thermique $C_i$ et pour chaque molécule du gaz de mélange ayant une conductibilité thermique $D_i$, pour des conditions identiques de température (température du flux 25 lors de la mesure de pression $P_r$, typiquement 20°C) et de pression (Pression de mesure $P_r$), on a $\dfrac{C_i - D_i}{C_i} \geq 20\%$ et $\dfrac{C_i - D_i}{D_i} \geq 20\%$, voir de préférence $\dfrac{C_i - D_i}{C_i} \geq 30\%$ et $\dfrac{C_i - D_i}{D_i} \geq 30\%$). Cette différence d'au moins 20 ou 30% intervient dans la précision du dispositif 1, plus elle est élevée, plus le gaz d'intérêt est discriminé et réduit le recours à l'amplification électronique ; ce seuil qui est de manière optimale fixé à 20 ou 30 % peut être aussi inférieur à 20% dans d'autres modes de réalisation, mais plus ce seuil va être bas, moins bonne va être la résolution du dispositif selon l'invention ou plus il va falloir une électronique performante pour la discrimination, ou d'autres moyens techniques de réalisation redondants du dispositif décrit dans d'autres échelles de mesure;.

Au sein du dispositif 1, ledit chemin d'aspiration se rétrécit de manière localisée au niveau d'un trou de mesure 14. Le trou de mesure 14 est un trou pratiqué dans une plaque 15. La plaque 15 est typiquement en inox. La plaque 15 est amovible de manière à pouvoir la remplacer typiquement soit en cas d'usure du trou 14 soit pour changer de taille de trou 14 au sein du dispositif 1. Le trou 14 est de dimension connue typiquement de 5 μm à 150 μm de diamètre. L'écoulement passe par un deuxième trou 21 de diamètre supérieur (typiquement de l'ordre de 2mm) au trou de mesure 14. L'épaisseur de cette plaque perforée 15 est un élément d'ajustement de la perte de charge recherchée, et est très inférieure à la taille de l'orifice 14 micro-perforé (typiquement environ 10 fois inférieur)

Ce trou 14 est le passage de plus faible aire d'ouverture (par unité de surface perpendiculaire à la direction du flux 25) pour le flux de gaz 25 dans le dispositif 1 comparé au reste de l'ensemble du chemin d'aspiration, et même de préférence du chemin d'expiration et du chemin de dilution. Typiquement, tous les endroits du chemin d'aspiration (et même de préférence du chemin d'expiration et du chemin de dilution), à l'exception du trou 14 lui-même évidemment, ont une aire d'ouverture (par unité de surface perpendiculaire à la direction du flux 25) au moins 5 fois plus grande que l'aire d'ouverture (par unité de surface perpendiculaire à la direction du flux 25) du trou 14.

Le trou 14 est de forme circulaire.

L'au moins un capteur de pression 5, 6 comprend un premier capteur 6 de pression (dit d'aspiration) agencé pour mesurer une pression $P_r$ (plus exactement une dépression, directement liée à la force d'aspiration de la turbine 3) du gaz à analyser le long du chemin d'aspiration, de préférence mais de manière non limitative comprise entre 20 et 500 mbar ou plus large (comprise entre 4 et 500 mbar ou comprise entre 4 et 1000 mbar ou plus large selon les capacités de la turbine 3).

Le débitmètre massique 4 est agencé pour mesurer le paramètre représentatif du débit massique du gaz à analyser le long du chemin d'aspiration.

Les moyens de calcul 7 sont agencés pour quantifier la présence d'un gaz d'intérêt au sein du gaz à analyser (cette présence quantifiée étant typiquement une proportion en % de gaz d'intérêt dans le gaz à analyser ou en mol par litre de gaz à analyser ou sous la forme d'un volume de gaz d'intérêt par exemple en millilitre), à partir d'une mesure du paramètre représentatif du débit massique du gaz à analyser.

Les moyens de calcul 7 sont agencés pour quantifier la présence du gaz d'intérêt sous la forme d'un calcul d'une proportion ou d'un volume du gaz d'intérêt qui dépend du diamètre du trou 14 de mesure. Autrement dit, si l'on change le diamètre ou la largeur du trou 14 sans l'indiquer (par un programme, une commande, un bouton de réglage, etc...) au dispositif 1, le calcul de proportion ou de volume du gaz d'intérêt par le dispositif 1 devient faux.

Le premier capteur 6 de pression d'aspiration est situé le long du chemin d'aspiration entre l'orifice 2 et le trou de mesure 14, pour une meilleure précision de mesure.

Le débitmètre massique 4 est situé le long du chemin d'aspiration de sorte que le trou de mesure 14 soit situé le long du chemin d'aspiration entre l'orifice 2 et le débitmètre massique 4.

De manière expérimentale, les inventeurs de la présente invention se sont rendu compte qu'une excellente précision de mesure de la taille d'un trou (par exemple de référence 14 ou 22) de rétrécissement au sein d'un écoulement pouvait être atteinte en faisant passer le flux de gaz 25 (typiquement d'air) par ce trou et en mesurant le diamètre de ce trou $\phi_{cal}$ au moyen de la formule suivante :

$$\phi_{cal} = X \sqrt[4]{\dfrac{D_m^2}{P_r}} + Y = X \dfrac{\sqrt[2]{D_m}}{\sqrt[4]{P_r}} + Y$$

(ci après nommée « 1ère formule ») Avec $D_m$ le paramètre

représentatif du débit massique de ce flux de gaz à travers ce trou et $P_r$ la pression de ce flux de gaz, et X et Y des coefficients numériques de calibration.

Pour ce qui est de la mesure de $D_m$, le débitmètre massique 4 est optimisé pour un ou plusieurs types de gaz ayant une valeur par défaut de conductibilité thermique (aussi appelée conductivité thermique). Pour des gaz ayant une conductibilité thermique se démarquant de cette valeur par défaut, un facteur de correction est à appliquer.

Par exemple, dans le cas du débitmètre massique 4 de marque Honeywell de la série AWM, le débit $D_m$ mesuré par ce débitmètre 4 est à multiplier par un facteur 1 (pas de correction) si le flux de gaz est de l'air et/ou $N_2$ et/ou $O_2$ et/ou NO et/ou CO et est à corriger en le multipliant par un facteur de correction $K_{cal}=1,35$ si le flux de gaz est un flux de $CO_2$ et/ou $N_2O$ et/ou $NO_2$, ou un facteur $K_{cal}=0,5$ pour He, $K_{cal}=0,7$ pour $H_2$, $K_{cal}=0,95$ pour Ar, et $K_{cal}=1,1$ pour $CH_4$ et/ou $NH_3$, etc... (se reporter de manière générale à la notice du modèle de débitmètre 4 utilisé).

Considérons comme gaz à analyser un mélange d'$O_2$ et de $CO_2$ provenant de l'échantillon 13 et circulant dans le dispositif 1 le long du chemin d'aspiration ; supposons que l'on sait que ces deux gaz composent le mélange avec chacun une proportion de 0 à 100%, mais que les proportions de ces deux gaz sont inconnues. Considérons le cas où le diamètre réel $\phi_r$ du trou 14 est de 100 $\mu$m.

Si les moyens de calcul 7 calculent, par la 1ère formule précédemment décrite, un diamètre du trou 14 $\phi_{cal}$ de 100 $\mu$m, les moyens de calcul 7 en déduisent soit que la proportion d'$O_2$ dans le mélange est de 100%, soit que la proportion de $CO_2$ dans le mélange est de 0%, selon lequel de ces gaz est considéré comme étant le gaz d'intérêt.

Si les moyens de calcul 7 calculent, par la formule de $\phi_{cal}$ précédemment décrite, un diamètre du trou 14 de 135 $\mu$m, les moyens de calcul 7 en déduisent soit que la proportion d'$O_2$ dans le mélange est de 0%, soit que la proportion de $CO_2$ dans le mélange est de 100%, selon lequel de ces gaz est considéré comme étant le gaz d'intérêt.

De manière générale, si les moyens de calcul 7 calculent, par la formule de $\phi_{cal}$ précédemment décrite, un diamètre du trou 14 de $\phi_{cal}$, les moyens de calcul en déduisent (par une formule ci après nommée « 2ème formule ») que la proportion de $CO_2$ dans le mélange est de $\dfrac{\phi_{cal} - \phi_r}{K_{cal} - 1}$ % ou que la proportion d'$O_2$ dans le mélange est de $100 - \dfrac{\phi_{cal} - \phi_r}{K_{cal} - 1}$ % selon le gaz d'intérêt considéré, avec Kcal le facteur de correction du gaz d'intérêt comme expliqué précédemment (Kcal=1,35 dans le cas du $CO_2$).

Les moyens de calcul 7 ne sont pas obligés de passer par deux étapes de calcul du diamètre du trou 14 (1ère étape, 1ère formule) puis de déduction de la proportion du gaz d'intérêt (2ème étape, 2ème formule), mais peuvent directement calculer cette proportion en un seul calcul combinant les deux étapes et donc les deux formules.

Les moyens de calcul 7 sont donc agencés pour quantifier la présence du gaz d'intérêt sous la forme d'un calcul d'une proportion ou d'un volume du gaz d'intérêt qui dépend de préférence de manière affine de la racine carrée du paramètre $D_m$ représentatif du débit massique.

Eventuellement, dans le cas moins précis d'un développement limité de la première formule à l'ordre un, les moyens de calcul 7 sont agencés pour quantifier la présence du gaz d'intérêt sous la forme d'un calcul d'une proportion ou d'un volume du gaz d'intérêt qui dépend de manière affine du paramètre représentatif du débit massique.

De manière générale, dans le cas d'un développement limité de la première formule à l'ordre Z (avec Z un entier supérieur ou égal à 1), les moyens de calcul 7 sont agencés pour quantifier la présence du gaz d'intérêt sous la forme d'un polynôme de degré Z du paramètre représentatif du débit massique.

Dans ce contexte, deux variantes de l'invention, éventuellement combinables au sein d'un même dispositif 1, sont envisageables.

Dans une première variante, les moyens de calcul 7 sont agencés pour quantifier la présence du gaz d'intérêt en outre à partir d'une mesure de pression $P_r$ par le capteur de pression d'aspiration :

- de manière préférentielle, les moyens de calcul 7 sont agencés pour quantifier la présence du gaz d'intérêt sous la forme d'un calcul d'une proportion ou d'un volume du gaz d'intérêt qui dépend de manière affine de l'inverse de la racine quatrième de la mesure de pression par le capteur de pression d'aspiration. La proportion ou le volume du gaz d'intérêt est typiquement calculée selon la formule :

$$A \frac{\sqrt[2]{D_m}}{\sqrt[4]{P_r}} + B$$

avec $D_m$ le paramètre représentatif du débit massique mesuré par le débitmètre 4, $P_r$ la pression mesurée par le capteur 6 de pression d'aspiration, et A et B des coefficients numériques de calibration.

- Eventuellement, on peut faire des approximations. Par exemple, les moyens de calcul 7 peuvent être agencés pour quantifier la présence du gaz d'intérêt sous la forme d'un calcul d'une proportion ou d'un volume du gaz d'intérêt qui dépend de manière affine de l'inverse de la mesure de pression par le capteur 6 de pression d'aspiration. La proportion ou le volume du gaz d'intérêt est typiquement calculée selon la formule :

$$M \frac{D_m}{P_r} + N$$

avec $D_m$ le paramètre représentatif du débit massique mesuré par le débitmètre 4, $P_r$ la pression mesurée par le capteur 6 de pression d'aspiration, M et N des coefficients numériques de calibration.

Dans une deuxième variante, $P_r$ la pression mesurée par le capteur 6 de pression d'aspiration n'est pas pris en compte dans la formule de calcul de la proportion ou du volume de gaz d'intérêt, mais sert de déclencheur (« trigger ») : les moyens de calcul 7 sont agencés pour déclencher une quantification de la présence du gaz d'intérêt pour une valeur de la pression mesurée $P_r$ par le capteur 6 de pression d'aspiration correspondant à la valeur de référence de pression d'aspiration, les moyens de calcul 7 étant agencés pour quantifier la présence du gaz d'intérêt à partir d'une valeur $D_m$ du paramètre représentatif du débit massique mesurée simultanément à la mesure de pression mesurant la valeur de pression correspondant à la valeur de référence de pression d'aspiration. Les moyens de calcul 7 sont alors agencés pour quantifier la présence du gaz d'intérêt sous la forme d'un calcul d'une proportion ou d'un volume du gaz d'intérêt :

- De préférence selon la formule :

$$A^* \sqrt[2]{D_m} + B$$

avec $D_m$ le paramètre représentatif du débit massique mesuré par le débitmètre 4, et A* et B des coefficients numériques de calibration.
- Eventuellement selon la formule :
$M^*D_m + N$ ou tout autre polynôme de degré Z de $D_m$ comme expliqué précédemment, avec $D_m$ le paramètre représentatif du débit massique mesuré par le débitmètre 4, et M* et N des coefficients numériques de calibration.

Selon l'invention, tous les facteurs de calibration A, B, M, N, A*, M*, a, b, a* sont mémorisés par les moyens de calcul 7 et sont définis par avance, typiquement en calibrant le dispositif 1 avec des échantillons 13 aux proportions connues de différents gaz ou avec des échantillons 13 munis chacun d'un trou de fuite 22 de dimension connue.

La valeur de chaque facteur de calibration dépend du gaz considéré. Par exemple, on peut présupposer un gaz de mélange d'$O_2$ mélangé avec un gaz d'intérêt de $CO_2$, ou un gaz de mélange d'He mélangé avec un gaz d'intérêt de $CH_4+NH_3$, etc...

Le dispositif 1 comprend donc une interface agencée pour définir le gaz de mélange et le gaz d'intérêt, et les moyens de calcul 7 sont agencés pour sélectionner les valeurs des facteurs de calibration en fonction des gaz de mélange et d'intérêt définis.

Le chemin d'aspiration passe successivement par l'orifice 2, le filtre 23, le capteur 5 de pression, la vanne 8, le capteur de pression 6, le trou de mesure 14, le capteur 20 de gaz, le trou de passage 21, le débitmètre 4, les moyens de génération 3 et la vanne 16.

Le dispositif 1 comprend en outre au moins un capteur 20 agencé pour quantifier la présence d'un gaz constituée d'une molécule donnée dont la conductibilité thermique ne serait pas discriminée par un autre gaz ou molécule présent(e).

Les moyens de calcul 7 (par exemple dans un cas où le gaz de mélange est de l'$O_2$ et où le gaz d'intérêt est un mélange $CO_2 + NO_2$) sont en outre agencés pour quantifier la présence d'une première molécule d'intérêt (par exemple $CO_2$ dans ce cas) du gaz d'intérêt ayant une certaine conductibilité thermique, le dispositif 1 comprenant pour cela le long du chemin d'aspiration au moins un capteur 20 de gaz (par exemple capteur de $NO_2$ dans ce cas, par exemple de marque City technology) agencé pour quantifier la présence (proportion en % ou en $mol.l^{-1}$ ou volume par exemple) d'au moins une autre molécule d'intérêt (par exemple $NO_2$ dans ce cas) qui a une conductibilité thermique différente au plus de 10% par rapport à la conductibilité thermique de la première molécule d'intérêt pour des conditions de pression et de température identiques, les moyens de calcul 7 étant agencés pour quantifier la présence de la première molécule d'intérêt ($CO_2$) à partir (simple soustraction) d'une quantification de la présence du gaz d'intérêt ($CO_2+NO_2$) et d'une quantification de la présence des autres molécules d'intérêt ($NO_2$).

Par exemple si on mesure :

Proportion du gaz d'intérêt $CO_2$+ $NO_2$=20% du gaz à analyser
Proportion $NO_2$= 5% du gaz à analyser

Alors on en déduit :

Proportion du gaz de mélange ($O_2$) = 100 - Proportion $CO_2$+$NO_2$=80% du gaz à analyser
Proportion $CO_2$= 15% du gaz à analyser

Le capteur 20 est situé le long du chemin d'aspiration de sorte que le trou de mesure 14 soit situé entre l'orifice 2 et le capteur 20. Le capteur 20 est situé dans une chambre de mesure le long du chemin d'aspiration entre le trou de mesure 14 et un trou de passage 21 plus large que le trou 14 de mesure.

L'au moins un capteur 20 peut aussi être un capteur d'$O_2$, ou autre (par exemple un assemblage capteur d'$O_2$ et capteur de $NO_2$), par exemple si le gaz de mélange comprend un mélange d'$O_2$ et de $N_2$ ceci afin de discriminer ces deux molécules.

Chemin de dilution

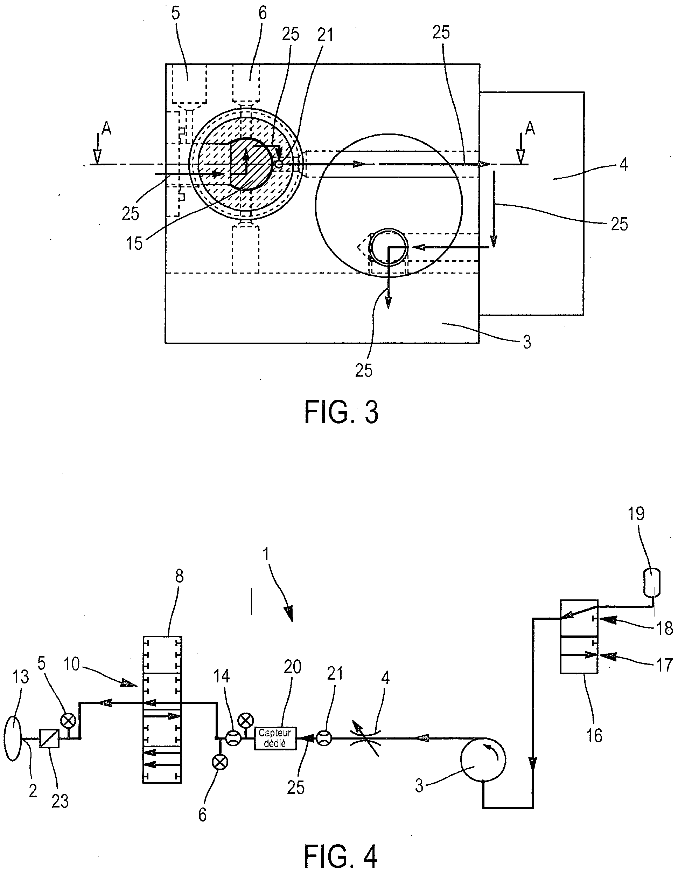

En référence à la figure 4, pour la même position (deuxième position 10) de la vanne 8 que le chemin d'aspiration, et pour les moyens de génération 3 expirant le flux de gaz 25, les moyens 3 pour générer le flux de gaz 25 sont agencés pour expirer un gaz de dilution le long du chemin de dilution.

Le chemin de dilution correspond donc au chemin d'aspiration mais parcouru par le flux de gaz 25 en sens inverse.

Pour le chemin de dilution, la vanne 16 est dans sa deuxième position 18 reliant les moyens 3 à la source 19 de gaz. Le gaz de solution est donc le gaz de référence de la source 19 (qui est typiquement une cartouche de gaz).

Le chemin de dilution sert à augmenter le volume du gaz à analyser de l'échantillon 13.

Chemin de dilution : exemple 1

Imaginons que l'échantillon 13 ne contienne initialement comme gaz initial qu'un mélange de $CO_2$ + $NO_2$ sans $O_2$, mais en trop faible quantité pour pouvoir aspirer ce mélange dans le dispositif 1 en remplissant tout le chemin d'aspiration : il est alors impossible de déterminer les proportions de $CO_2$ et de $NO_2$ en l'état. Par contre, si par le chemin de dilution, on insère dans l'échantillon 13 de l'$O_2$ provenant de la source 19, alors l'échantillon 13 contient un mélange de $CO_2$ + $NO_2$ + $O_2$ dans une quantité suffisante pour faire des mesures. On peut déterminer la proportion de $CO_2$, $NO_2$, et $O_2$ après dilution comme décrit précédemment. On peut alors en déduire la proportion de $CO_2$ et de $NO_2$ avant la dilution.

Par exemple si on mesure :

Proportion $CO_2$+$NO_2$=20% du gaz à analyser après dilution

Proportion $NO_2$= 5% du gaz à analyser après dilution

Alors on en déduit :

Proportion $O_2$=100 - Proportion $CO_2$+$NO_2$=80% du gaz à analyser après dilution

Proportion $CO_2$= 15% du gaz à analyser après dilution

Soit :

Proportion $NO_2$= 25% du gaz initial avant dilution

Proportion $CO_2$= 75% du gaz initial avant dilution

Chemin de dilution : exemple 2

Imaginons que l'échantillon 13 ne contienne initialement comme gaz initial qu'un mélange de $N_2$ et d'$O_2$, mais en trop faible quantité pour pouvoir aspirer ce mélange dans le dispositif 1 en remplissant tout le chemin d'aspiration : il est alors impossible de déterminer les proportions ou volumes d'$O_2$ et de $N_2$ en l'état. Par contre, si par le chemin de dilution, on insère dans l'échantillon

13 du $CO_2$ provenant de la source 19, alors l'échantillon 13 contient un mélange de $CO_2$ + $N_2$ + $O_2$ dans une quantité suffisante pour faire des mesures. On peut déterminer la proportion de $CO_2$, $N_2$, et $O_2$ après dilution comme décrit précédemment en utilisant le volume de gaz injecté et le volume de gaz aspiré. On peut alors en déduire la proportion de $O_2$ et de $N_2$ avant la dilution :

- Stade initial : Le volume de gaz supposé contenu est V1 (typiquement cette problématique se rencontre pour des étuis dont le volume disponible est inférieur à 3ml). Ce volume V1 est inconnu au stade initial. Le volume V1 contient majoritairement du N2 et des traces d'O2 non mesurables du fait du volume de gaz disponible dans le contenant.

- Dilution : on procède à une dilution du volume avec 100% de CO2 par injection d'un volume V2 = 10 ml au moins suffisant pour exciter le capteur d'02 (repère 20). Le volume V2 est ensuite ré-aspiré.

Les proportions données sont :

Mélange O2+CO2+N2 = 1.34 au lieu de 1.35 (référence N2+02, air)

La quantité de N2+O2 présent dans le mélange dilué est = (100-1.34x100/1.35)xV2 = 0.00296 x V2=0.0296ml

Le volume de CO2 contenu dans V2 est V2-0.037%xV2 = 9.97ml

La concentration en CO2 dans V2 est devenue 99.704%

La proportion de 02 dans le mélange dilué V2 est donné par le capteur 20 = 0.01%, soit 0.001 ml

La proportion d'02 dans le volume V1 initial est = 0.001x10/0.0296 = 3.378%

Et on peut en déduire le volume V1: (100-99.704)*10ml = 2.96 ml

Chemin d'expiration

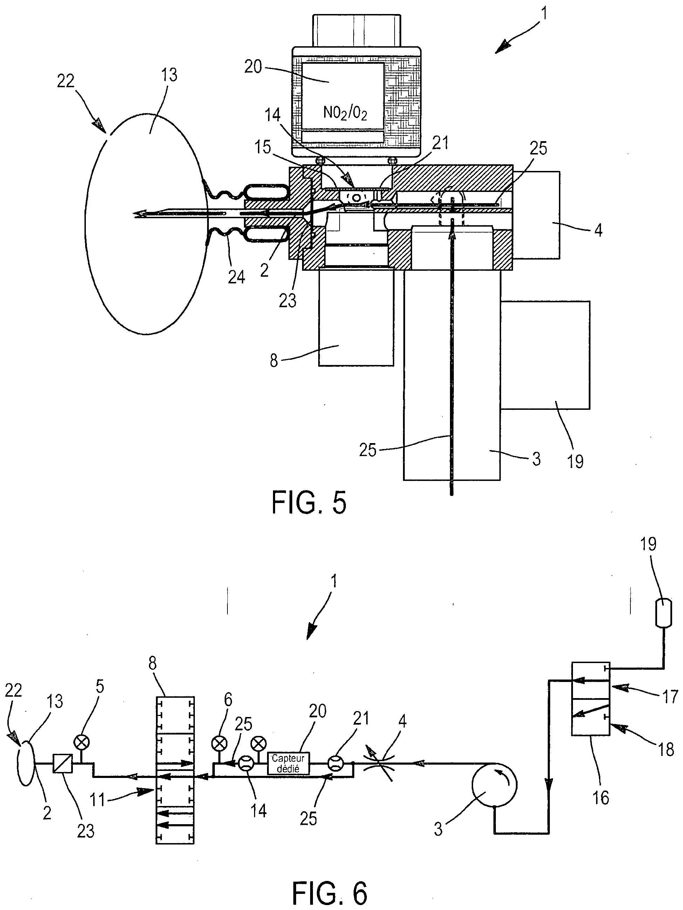

En référence aux figures 5 et 6, pour une troisième position 11 de la vanne 8, et pour les moyens de génération 3 expirant le flux de gaz 25, les moyens 3 pour générer le flux de gaz sont agencés pour expirer un gaz de fuite le long du chemin d'expiration.

Selon la position de la vanne 16, le gaz de fuite (de préférence de l'$O_2$ ou de l'air) provient de l'atmosphère extérieure ou de la source 19.

L'au moins un capteur de pression comprend un capteur 5 de pression d'expiration agencé pour mesurer une pression $P_r$ du gaz de fuite le long du chemin d'expiration, de préférence mais de manière non limitative comprise entre 20 et 500 mbar ou plus large comprise entre 4 et 500 mbar ou comprise entre 4 et 1000 mbar, et dans tous les cas, dans les limites de la perte de charge du circuit pneumatique et de la résistance à la pression des organes constituant l'invention.

Le débitmètre massique 4 est agencé pour mesurer un paramètre représentatif du débit massique du gaz de fuite le long du chemin d'expiration.

Dans un mode de réalisation en dehors de la portée de la présente invention, les moyens de calcul 7 sont agencés pour déterminer la taille d'un trou de fuite 22 de l'échantillon 13 (dans lequel est inséré le gaz de fuite expiré par le dispositif 1), à partir d'une mesure du paramètre représentatif du débit massique.

Le capteur 5 de pression d'expiration est situé le long du chemin d'expiration entre le débitmètre 4 et l'orifice 2.

Les moyens de calcul 7 sont agencés pour déterminer la taille du trou de fuite 22 de préférence sous la forme d'un calcul qui dépend de manière affine de la racine carrée du paramètre représentatif du débit massique (cf première formule décrite précédemment).

Eventuellement, dans le cas moins précis d'un développement limité de la première formule à l'ordre un, les moyens de calcul 7 sont agencés pour déterminer la taille du trou de fuite 22 sous la forme d'un calcul qui dépend de manière affine du paramètre représentatif du débit massique.

De manière générale, dans le cas d'un développement limité de la première formule à l'ordre Z (avec Z un entier supérieur ou égal à 1), les moyens de calcul 7 sont agencés pour déterminer la taille du trou de fuite 22 sous la forme d'un polynôme de degré Z du paramètre représentatif du débit massique

Dans ce contexte, deux variantes, éventuellement combinables au sein d'un même dispositif 1, sont envisageables.

Dans une première variante, les moyens de calcul 7 sont agencés pour déterminer la taille du trou 22 en outre à partir d'une mesure de pression par le capteur 5 de pression d'expiration, par exemple sous la forme d'un calcul qui dépend de préférence de manière affine de l'inverse de la racine quatrième de la mesure de pression. Typiquement, les moyens de calcul 7 sont agencés pour déterminer la taille du trou 22 selon la formule :

$$a \frac{\sqrt[2]{D_m}}{\sqrt[4]{P_r}} + b$$

avec $D_m$ le paramètre représentatif du débit massique mesuré par le débitmètre 4, $P_r$ la pression mesurée par le capteur 5 de pression d'expiration, et a et b des coefficients numériques de calibration.

Selon l'invention, on peut alors mesurer des diamètres de trou de fuite 22 typiquement jusqu'à un minimum de 0,05 µm.

Dans une deuxième variante, $P_r$ la pression mesurée par le capteur 5 de pression d'expiration n'est pas pris en compte dans la formule de calcul de la taille du trou de fuite 22, mais sert de déclencheur (« trigger ») : les moyens de calcul 7 sont agencés pour déclencher une détermination de la taille du trou 22 pour une valeur de la pression mesurée par le capteur 5 de pression d'expiration correspondant à une valeur de référence de pression d'expiration, les moyens de calcul 7 étant agencés pour déterminer la taille du trou 22 à partir d'une valeur du paramètre représentatif du débit massique $D_m$ mesurée simultanément à la mesure de pression mesurant la valeur de pression correspondant à la valeur de référence de pression d'expiration. Les moyens de calcul 7 sont par exemple agencés pour déterminer la taille du trou 22 selon la formule :

$$a^* \sqrt[2]{D_m} + b$$

avec $D_m$ le paramètre représentatif du débit massique mesuré par le débitmètre 4, et $a^*$ et b des coefficients numériques de calibration.

Le chemin d'expiration se scinde en deux parties qui se séparent avant le trou de mesure 14 et qui se rejoignent après le trou de mesure 14 :

- une première partie passe par le trou 14 de mesure, (et comprend le capteur 6 et la chambre de mesure comprise entre le trou de mesure 14 et le trou de passage 21)
- une deuxième partie ne passe pas par le trou de mesure 14, pour que le trou de mesure 14 ne limite pas le débit du flux de gaz 25 expiré dans le chemin d'expiration.

Le chemin d'expiration passe donc successivement par la vanne 16, les moyens de génération 3, le débitmètre 4, les deux parties qui se séparent avant le trou de mesure 14 et qui se rejoignent après le trou 14 de mesure, la vanne 8, le capteur 5 de pression, le filtre 23, et l'orifice 2.

Chemin de calibration

L'au moins un chemin d'écoulement comprend un chemin de calibration passant par l'orifice 2, qui correspond au chemin d'aspiration ou de dilution. Ce chemin de calibration se rétrécit de manière localisée au niveau du trou 14 de mesure. Lorsque les moyens 3 de génération génèrent un flux de gaz 25 (gaz de calibration) en aspiration ou en expiration dans ce chemin de calibration sans que l'orifice 2 ne soit connecté à un échantillon fermé 13 (l'orifice 2 débouchant plutôt de préférence à l'air libre), les moyens de calcul 7 sont agencés pour :

1) déterminer la taille du trou 14 de mesure à partir d'une mesure du paramètre représentatif du débit massique $D_m$ par le débitmètre 4, sur le même principe que la détermination de la taille d'un trou de fuite 22 précédemment décrite, et
2) ajuster ses coefficients numériques (typiquement a, b, a*, etc.) pour le calcul d'une taille d'un trou 22 de fuite si sa détermination de la taille $\phi_{cal}$ du trou 14 de mesure ne correspond pas à la taille réelle $\phi_r$ du trou de mesure 14 mémorisée par les moyens 7 de calcul,
3) Et optionnellement ré-itérer les étapes 1) et 2) si dessus jusqu'à ce que la détermination de la taille du trou 14 de mesure corresponde à un pourcentage d'erreur près à la taille réelle du trou de mesure 14 mémorisée par les moyens de calcul.

Chemin de court-circuit

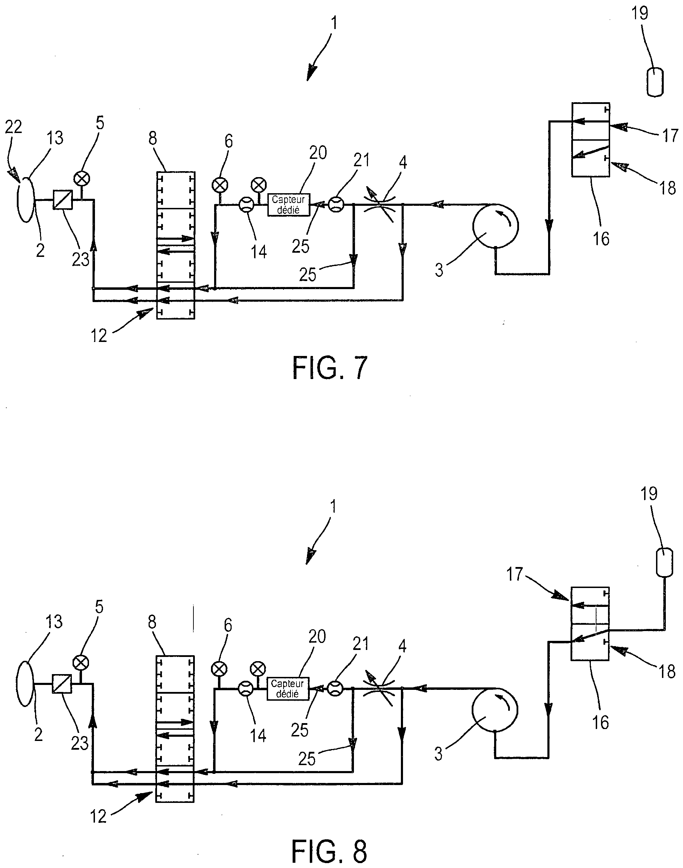

En référence aux figures 7 et 8 représentant des modes de réalisation en dehors de la portée de la présente invention, la vanne 8 dans sa quatrième position 12 est agencée pour compléter le chemin d'expiration par un chemin de court-circuit passant par l'orifice 2 et les moyens 3 de génération de flux mais ne passant pas par le débitmètre 4 (ce chemin de court-circuit ne faisant donc pas partie des chemins d'écoulement tel que définis précédemment). La vanne 8 est agencée pour ajuster le débit passant au total par le chemin d'expiration et le chemin de court-circuit. Cela permet des débits $D_m$ plus grand, et donc de mesurer d'autres échelles de diamètre de trou de fuite 22 ou de gonfler rapidement l'échantillon 13 pour tester sa solidité jusqu'à éclatement par phénomène de fatigue ou de stress successifs. Les moyens de calcul 7 sont agencés pour déduire, à partir d'une mesure de débit $D_m$ le long du chemin d'expiration par le débitmètre massique 4, le débit passant au total par le chemin d'expiration et le chemin de court-circuit lorsque la vanne 8 ouvre ce chemin de court-circuit.

Typiquement, les moyens de calcul 7 appliquent une simple multiplication, par un coefficient de calibration, du débit $D_m$ mesuré par le débitmètre massique 4 pour obtenir le débit passant au total par le chemin d'expiration et le chemin de court-circuit lorsque la vanne 8 ouvre ce chemin de court-circuit. Ou bien de préférence, les moyens 7 de calcul modifient la valeur des coefficients de calibration a et a* lors de la détermination de la taille du trou de fuite 22, pour tenir compte du fait que le débit passant au total par le chemin d'expiration et le chemin de court-circuit est plus grand que la mesure, par le débitmètre 4, du paramètre $D_m$ représentatif du débit massique du gaz de fuite le long du chemin d'expiration.

On va maintenant décrire un exemple de procédé dont la séquence peut être modifiée selon l'invention mis en oeuvre par le dispositif 1 des figures 1 à 8. Les types de gaz cités ($O_2$, $CO_2$, $NO_2$ etc..) ne sont qu'illustratifs et peuvent bien évidemment varier.

Dilution

Tout d'abord, avant l'aspiration du gaz à analyser, le procédé selon l'invention comprend une expiration (par les moyens 3) du gaz de dilution ($CO_2$ provenant de la source 19) s'écoulant le long du chemin de dilution jusque dans l'échantillon 13 comprenant un gaz initial (mélange de $CO_2$ et $NO_2$) qui comprend de préférence mais pas nécessairement le gaz d'intérêt.

Analyse de gaz

Après la dilution, le procédé selon l'invention comprend une aspiration (par les moyens 3) du gaz à analyser ($O_2$ + $CO_2$ + $NO_2$) en provenance de l'échantillon 13, ledit gaz à analyser aspiré s'écoulant le long du chemin d'aspiration débutant par l'orifice 2 et se rétrécissant de manière localisée au niveau du trou de mesure 14.

Pendant l'aspiration, le procédé selon l'invention comprend simultanément :

- une mesure de pression (plus exactement une dépression de l'aspiration, a priori négative mais considérée en valeur absolue pour les calculs) $P_r$ du gaz à analyser le long du chemin d'aspiration par le capteur 6, de préférence mais de manière non limitative comprise entre -20 et -500 mbar ou plus large comprise entre 4 et 500 mbar ou comprise entre 4 et 1000 mbar ou plus selon la capacité de la turbine 3 ;
- une mesure d'un paramètre représentatif du débit massique du gaz à analyser le long du chemin d'aspiration, par le débitmètre 4.

Le procédé selon l'invention comprend ensuite une quantification, par les moyens de calcul 7, de la présence du gaz d'intérêt ($CO_2$ + $NO_2$) au sein du gaz à analyser ($O_2$ + $CO_2$ + $NO_2$) à partir de cette dernière mesure du paramètre représentatif du débit massique : par exemple proportion $CO_2$+ $NO_2$=20% du gaz à analyser après dilution. La quantification de la présence du gaz d'intérêt comprend un calcul tel que décrit pour la description du dispositif 1.

Le gaz d'intérêt ($CO_2$ + $NO_2$) comprend de 0 à 100% d'une première molécule d'intérêt ($CO_2$) ayant une certaine conductibilité thermique, et de 0 à 100% d'autres molécules d'intérêt ($NO_2$) qui ont une conductibilité thermique différente au plus de 10% par rapport à la conductibilité thermique de la première molécule d'intérêt pour des conditions identiques de température et de pression.

Le procédé selon l'invention comprend en outre (simultanément à la mesure de pression et du paramètre représentatif du débit massique) une quantification de la présence des autres molécules d'intérêt ($NO_2$) au sein du gaz à analyser ($O_2$ + $CO_2$ + $NO_2$) au moyen du capteur 20 : par exemple proportion $NO_2$= 5% du gaz à analyser après dilution.

Le procédé selon l'invention comprend en outre une quantification de la présence de la première molécule d'intérêt ($CO_2$) dans le gaz à analyser ($O_2$ + $CO_2$ + $NO_2$) à partir de la quantification de la présence du gaz d'intérêt ($CO_2$ + $NO_2$) au sein du gaz à analyser ($O_2$ + $CO_2$ + $NO_2$) et de la quantification de la présence des autres molécules d'intérêt ($NO_2$) au sein du gaz à analyser ($O_2$ + $CO_2$ + $NO_2$) : par exemple proportion $CO_2$= 15% du gaz à analyser après dilution.

Le procédé selon l'invention comprend en outre une quantification de la présence de la première molé-

cule d'intérêt ($CO_2$) dans le gaz initial ($CO_2$ + $NO_2$) à partir de la quantification de la présence de la première molécule d'intérêt ($CO_2$) au sein du gaz à analyser ($O_2$ + $CO_2$ + $NO_2$) et de la quantification de la présence des autres molécules d'intérêt ($NO_2$) au sein du gaz à analyser ($O_2$ + $CO_2$ + $NO_2$) : par exemple proportion $CO_2$= 75% du gaz initial.

Le procédé selon l'invention comprend en outre une quantification de la présence des autres molécules d'intérêt ($NO_2$) dans le gaz initial ($CO_2$ + $NO_2$) à partir de la quantification de la présence de la première molécule d'intérêt ($CO_2$) au sein du gaz à analyser ($O_2$ + $CO_2$ + $NO_2$) et de la quantification de la présence des autres molécules d'intérêt ($NO_2$) au sein du gaz à analyser ($O_2$ + $CO_2$ + $NO_2$) : par exemple proportion $NO_2$= 25% du gaz initial.

Ensuite, vient le test mécanique de l'échantillon 13, qui est en dehors de la portée de la présente invention.

Calibration de mesure de fuite

Ce procédé comprend alors un écoulement (généré par les moyens 3) d'un gaz de calibration (de préférence de l'air extérieur ou le gaz de la source 19) le long du chemin de calibration, et, simultanément à cet écoulement :

1) une mesure de pression $P_r$ du gaz de calibration le long du chemin de calibration par le capteur 5 ou 6, de préférence mais de manière non limitative comprise entre 20 et 500 mbar ou plus large comprise entre 4 et 500 mbar ou comprise entre 4 et 1000 mbar
2) une mesure d'un paramètre représentatif du débit massique du gaz de calibration le long du chemin de calibration, par le débitmètre 4
3) une détermination de la taille du trou de mesure 14 à partir de cette dernière mesure du paramètre représentatif du débit massique, par les moyens de calcul 7, et
4) un ajustement, par les moyens de calcul 7, de coefficients de calibration a, a*, b pour le calcul d'une taille d'un trou de fuite 22 si la détermination $\phi_{cal}$ de la taille du trou de mesure 14 ne correspond pas à une taille réelle $\phi_r$ du trou de mesure 14 mémorisée par les moyens de calcul, et
5) optionnellement une réitération des étapes 1 à 4 précédentes.

Mesure de fuite

Le procédé comprend ensuite une expiration du gaz de fuite (de préférence de l'air extérieur ou le gaz de la source 19 ou un gaz traceur permettant de localiser la fuite, colorant ou mesurable par d'autres moyens extérieurs) s'écoulant le long du chemin d'expiration se terminant par l'orifice 2.

Pendant l'expiration, le procédé comprend simultanément :

- une mesure de pression $P_r$ du gaz de fuite le long du chemin d'expiration par le capteur 5, de préférence mais de manière non limitative comprise entre 20 et 500 mbar ou plus large comprise entre 4 et 500 mbar ou comprise entre 4 et 1000 mbar, et dans tous les cas, dans les limites de la perte de charge du circuit pneumatique et de la résistance à la pression des organes constituant ce mode de réalisation en dehors de la portée de la présente invention.
- une mesure d'un paramètre représentatif du débit massique du gaz de fuite le long du chemin d'expiration, par le débitmètre 4.

Le procédé comprend ensuite une détermination, par les moyens 7 de calcul, de la taille du trou de fuite 22 dans l'échantillon 13, à partir de cette dernière mesure du paramètre représentatif du débit massique.

La détermination de la taille du trou de fuite 22 comprend un calcul tel que décrit pour la description du dispositif 1.

Si le trou de fuite 22 est trop grand, il faut augmenter le débit de gaz de fuite pour rechercher l'atteinte d'une pression de consigne. Le procédé selon l'invention comprend alors un ajustement, par la vanne 8 agencée pour compléter le chemin d'expiration par un chemin de court-circuit passant par l'orifice et les moyens de génération de flux mais ne passant pas par le débitmètre, du débit passant au total par le chemin d'expiration et le chemin de court-circuit, ladite vanne ouvrant le chemin de court circuit selon une ouverture de taille réglable.

Le procédé comprend alors une détermination, par les moyens 7, et à partir de la mesure de débit le long du chemin d'expiration, du débit passant au total par le chemin d'expiration et le chemin de court-circuit lorsque la vanne ouvre le chemin de court-circuit.

Plus concrètement, les moyens 7 de calcul modifient la valeur des coefficients de calibration a et a* lors de la détermination de la taille du trou de fuite 22, pour tenir compte que fait que le débit passant au total par le chemin d'expiration et le chemin de court-circuit est plus grand que la mesure, par le débitmètre 4, du paramètre $D_m$ représentatif du débit massique du gaz de fuite le long du chemin d'expiration.

Test de Résistance / d'Eclatement

Après avoir déterminé la taille du trou de fuite 22, le débit de gaz est poussé à une forte valeur, éventuellement à débit contrôlé pour tester l'éclatement de l'échantillon 13 dans une dynamique voulue, ce mode de réalisation étant en dehors de la portée de la présente invention.

On note quand dans ce procédé, l'échantillon 13 peut être soumis à des contraintes mécaniques externes comme une sur-enveloppe de bridage, la pression atmosphérique, une immersion dans un fluide, etc...

On remarque en outre que les différentes étapes de ce procédé peuvent être inversées, ou faites de manière simultanées ou être optionnelles. Par exemple, l'étape de calibration n'est pas nécessaire avant la mesure de fuite. De même, la mesure de fuite est complètement indépendante de l'analyse de gaz, et la mesure de fuite peut être réalisée avant l'analyse de gaz ou sans l'analyse de gaz. Dans un cas préférentiel, pour gagner du temps, la mesure de fuite peut être réalisée simultanément à la dilution de préférence une fois une pression d'équilibre atteinte, le gaz de dilution expiré jouant aussi le rôle de gaz de fuite expiré.

**Revendications**

1. Dispositif (1) de test d'un échantillon (13) par flux gazeux (25), comprenant :

   - un orifice (2),
   - des moyens (3) pour générer un flux de gaz (25) dans le dispositif le long d'au moins un chemin d'écoulement passant par l'o ri fi ce,
   - au moins un capteur de pression (5, 6), chaque capteur de pression étant agencé pour mesurer une pression du flux de gaz le long d'au moins un chemin d'écoulement, et
   - un débitmètre massique (4), agencé pour mesurer un paramètre représentatif du débit massique du flux de gaz le long de chaque chemin d'écoulement,

   dispositif dans lequel :

   - l'au moins un chemin d'écoulement comprend un chemin d'aspiration débutant par l'orifice,
   - les moyens pour générer le flux de gaz sont agencés pour aspirer un gaz à analyser de sorte que ce gaz à analyser s'écoule le long du chemin d'aspiration,
   - au sein du dispositif, ledit chemin d'aspiration se rétrécit de manière localisée au niveau d'un trou de mesure (14),
   - l'au moins un capteur de pression comprend un capteur de pression d'aspiration (6) agencé pour mesurer une pression du gaz à analyser le long du chemin d'aspiration,
   - le débitmètre massique est agencé pour mesurer un paramètre représentatif du débit massique du gaz à analyser le long du chemin d'aspiration, et
   - le dispositif comprend en outre des moyens de calcul (7) agencés pour quantifier la présence d'un gaz d'intérêt au sein du gaz à analyser, à partir d'une mesure du paramètre représentatif du débit massique du gaz à analyser le long du chemin d'aspiration,

et **caractérisé en ce que** les moyens de calcul sont agencés pour quantifier la présence du gaz d'intérêt sous la forme d'un calcul d'une proportion ou d'un volume du gaz d'intérêt qui dépend du diamètre du trou de mesure.

2. Dispositif selon la revendication 1, **caractérisé en ce que** le débitmètre massique est un débitmètre massique à conductibilité thermique.

3. Dispositif selon la revendication 1 ou 2, **caractérisé en ce que** les moyens de calcul sont agencés pour quantifier la présence du gaz d'intérêt sous la forme d'un calcul d'une proportion du gaz d'intérêt, et **en ce que** cette proportion est une proportion en pourcentage de gaz d'intérêt dans le gaz à analyser ou en mol par litre de gaz à analyser.

4. Dispositif selon l'une quelconque des revendications précédentes, **caractérisé en ce que** le capteur de pression d'aspiration est situé le long du chemin d'aspiration entre l'orifice et le trou de mesure.

5. Dispositif selon l'une quelconque des revendications précédentes, **caractérisé en ce que** le débitmètre massique est situé le long du chemin d'aspiration de sorte que le trou de mesure soit situé le long du chemin d'aspiration entre l'orifice et le débitmètre massique.

6. Dispositif selon l'une quelconque des revendications précédentes, **caractérisé en ce que** les moyens de calcul sont agencés pour quantifier la présence du gaz d'intérêt sous la forme d'un calcul d'une proportion ou d'un volume du gaz d'intérêt qui dépend de manière affine de la racine carrée du paramètre représentatif du débit massique le long du chemin d'aspiration.

7. Dispositif selon l'une quelconque des revendications 1 à 5, **caractérisé en ce que** les moyens de calcul sont agencés pour quantifier la présence du gaz d'intérêt sous la forme d'un calcul d'une proportion ou d'un volume du gaz d'intérêt qui dépend de manière affine du paramètre représentatif du débit massique le long du chemin d'aspiration.

8. Dispositif selon l'une quelconque des revendications précédentes, **caractérisé en ce que** les moyens de calcul sont agencés pour quantifier la présence du gaz d'intérêt en outre à partir d'une mesure de pression le long du chemin d'aspiration par le capteur de pression d'aspiration.

9. Dispositif selon la revendication 8 considérée comme dépendante de la revendication 6, **caractérisé en ce que** les moyens de calcul sont agencés pour quantifier la présence du gaz d'intérêt sous la forme d'un calcul d'une proportion ou d'un volume du gaz d'intérêt qui dépend de manière affine de l'inverse de la racine quatrième de la mesure de pression le long du chemin d'aspiration par le capteur de pression d'aspiration.

10. Dispositif selon la revendication 9, **caractérisé en ce que** les moyens de calcul sont agencés pour quantifier la présence du gaz d'intérêt sous la forme d'un calcul d'une proportion ou d'un volume du gaz d'intérêt selon la formule :

$$A \frac{\sqrt[2]{D_m}}{\sqrt[4]{P_r}} + B$$

Avec $D_m$ le paramètre représentatif du débit massique, $P_r$ la pression mesurée par le capteur de pression d'aspiration, et A et B des coefficients numériques de calibration.

11. Dispositif selon la revendication 8 considérée comme dépendante de la revendication 7, **caractérisé en ce que** les moyens de calcul sont agencés pour quantifier la présence du gaz d'intérêt sous la forme d'un calcul d'une proportion ou d'un volume du gaz d'intérêt qui dépend de manière affine de l'inverse de la mesure de pression le long du chemin d'aspiration par le capteur de pression d'aspiration.

12. Dispositif selon la revendication 11, **caractérisé en ce que** les moyens de calcul sont agencés pour quantifier la présence du gaz d'intérêt sous la forme d'un calcul d'une proportion ou d'un volume du gaz d'intérêt selon la formule :

$$M \frac{D_m}{P_r} + N$$

Avec $D_m$ le paramètre représentatif du débit massique, $P_r$ la pression mesurée par le capteur de pression d'aspiration, M et N des coefficients numériques de calibration.

13. Dispositif selon l'une quelconque des revendications 1 à 7, **caractérisé en ce que** les moyens de calcul sont agencés pour déclencher une quantification de la présence du gaz d'intérêt pour une valeur de la pression le long du chemin d'aspiration mesurée par le capteur de pression d'aspiration correspondant à une valeur de référence de pression d'aspiration, les moyens de calcul étant agencés pour quantifier la présence du gaz d'intérêt à partir d'une valeur du paramètre représentatif du débit massique le long du chemin d'aspiration mesurée simultanément à la mesure de pression mesurant la valeur de pression correspondant à la valeur de référence de pression d'aspiration.

14. Dispositif selon la revendication 13 considérée comme dépendante de la revendication 6, **caractérisé en ce que** les moyens de calcul sont agencés pour quantifier la présence du gaz d'intérêt sous la forme d'un calcul d'une proportion ou d'un volume du gaz d'intérêt selon la formule :

$$A^* \sqrt[2]{D_m} + B$$

Avec $D_m$ le paramètre représentatif du débit massique, et A* et B des coefficients numériques de calibration.

15. Dispositif selon la revendication 13 considérée comme dépendante de la revendication 7, **caractérisé en ce que** les moyens de calcul sont agencés pour quantifier la présence du gaz d'intérêt sous la forme d'un calcul d'une proportion ou d'un volume du gaz d'intérêt selon la formule :

$$M^* D_m + N$$

Avec $D_m$ le paramètre représentatif du débit massique, et M* et N des coefficients numériques de calibration.

16. Dispositif selon l'une quelconque des revendications précédentes, **caractérisé en ce que** les moyens de calcul sont en outre agencés pour quantifier la présence d'une première molécule d'intérêt du gaz d'intérêt, le dispositif comprenant en outre le long du chemin d'aspiration au moins un capteur (20) de gaz agencé pour quantifier la présence d'au moins une autre molécule d'intérêt du gaz d'intérêt, les moyens de calcul étant agencés pour quantifier la présence de la première molécule d'intérêt à partir d'une quantification de la présence du gaz d'intérêt et d'une quantification de la présence des autres molécules d'intérêt.

17. Dispositif selon l'une quelconque des revendications précédentes, **caractérisé en ce que** l'au moins un chemin d'écoulement comprend un chemin de dilution se terminant par l'orifice, les moyens pour générer le flux de gaz étant agencés pour expirer un gaz de dilution le long du chemin de dilution.

18. Procédé de test d'un échantillon par flux gazeux (25), **caractérisé en ce qu'**il comprend :

- une aspiration d'un gaz à analyser en provenance d'un échantillon (13), ledit gaz à analyser aspiré s'écoulant le long d'un chemin d'aspiration débutant par un orifice (2) relié à l'échantillon, ledit chemin d'aspiration se rétrécissant de manière localisée au niveau d'un trou de mesure (14),

- une mesure de pression du gaz à analyser le long du chemin d'aspiration,

- une mesure d'un paramètre représentatif du débit massique du gaz à analyser le long du chemin d'aspiration, et

- une quantification de la présence d'un gaz d'intérêt au sein du gaz à analyser, à partir de la mesure du paramètre représentatif du débit massique le long du chemin d'aspiration,

**caractérisé en ce que** la quantification de la présence du gaz d'intérêt comprend un calcul d'une proportion ou d'un volume du gaz d'intérêt qui dépend du diamètre du trou de mesure.

19. Procédé selon la revendication 18, **caractérisé en ce que** la mesure d'un paramètre représentatif du débit massique est une mesure par un débitmètre massique (4) à conductibilité thermique.

20. Procédé selon la revendication 18 ou 19, **caractérisé en ce que** la quantification de la présence du gaz d'intérêt comprend un calcul d'une proportion du gaz d'intérêt, et **en ce que** cette proportion est une proportion en pourcentage de gaz d'intérêt dans le gaz à analyser ou en mol par litre de gaz à analyser.

21. Procédé selon l'une quelconque des revendications 18 à 20, **caractérisé en ce que** la mesure de pression est effectuée par un capteur de pression d'aspiration (6) situé le long du chemin d'aspiration entre l'échantillon et le trou de mesure.

22. Procédé selon l'une quelconque des revendications 18 à 21, **caractérisé en ce que** la mesure du paramètre représentatif du débit massique est effectuée par un débitmètre massique situé le long du chemin d'aspiration de sorte que le trou de mesure soit situé le long du chemin d'aspiration entre l'échantillon et le débitmètre massique.

23. Procédé selon l'une quelconque des revendications 18 à 22, **caractérisé en ce que** la quantification de la présence du gaz d'intérêt comprend un calcul d'une proportion ou d'un volume du gaz d'intérêt qui dépend de manière affine de la racine carrée du paramètre représentatif du débit massique le long du chemin d'aspiration.

24. Procédé selon l'une quelconque des revendications 18 à 22, **caractérisé en ce que** la quantification de la présence du gaz d'intérêt comprend un calcul d'une proportion ou d'un volume du gaz d'intérêt qui dépend de manière affine du paramètre représentatif du débit massique le long du chemin d'aspiration.

25. Procédé selon l'une quelconque des revendications 18 à 24, **caractérisé en ce que** la quantification de la présence d'un gaz d'intérêt est effectuée en outre à partir de la pression mesurée le long du chemin d'aspiration.

26. Procédé selon la revendication 25 considérée comme dépendante de la revendication 23, **caractérisé en ce que** la quantification de la présence du gaz d'intérêt comprend un calcul d'une proportion ou d'un volume du gaz d'intérêt qui dépend de manière affine de l'inverse de la racine quatrième de la mesure de pression le long du chemin d'aspiration.

27. Procédé selon la revendication 26, **caractérisé en ce que** la quantification de la présence du gaz d'intérêt comprend un calcul d'une proportion ou d'un volume du gaz d'intérêt selon la formule :

$$A \frac{\sqrt[2]{D_m}}{\sqrt[4]{P_r}} + B$$

Avec $D_m$ le paramètre représentatif du débit massique, $P_r$ la pression mesurée, et A et B des coefficients numériques de calibration.

28. Procédé selon la revendication 25 considérée comme dépendante de la revendication 24, **caractérisé en ce que** la quantification de la présence du gaz d'intérêt comprend un calcul d'une proportion ou d'un volume du gaz d'intérêt qui dépend de manière affine de l'inverse de $P_r$ la pression mesurée le long du chemin d'aspiration.

29. Procédé selon la revendication 28, **caractérisé en ce que** la quantification de la présence du gaz d'intérêt comprend un calcul d'une proportion ou d'un volume du gaz d'intérêt selon la formule :

$$M \frac{D_m}{P_r} + N$$

Avec $D_m$ le paramètre représentatif du débit massique, $P_r$ la pression mesurée, M et N des coefficients numériques de calibration.

30. Procédé selon l'une quelconque des revendications 18 à 24, **caractérisé en ce que** la quantification de la présence d'un gaz d'intérêt est déclenchée pour une valeur de la pression mesuré le long du chemin d'aspiration correspondant à une valeur de référence de pression d'aspiration, la quantification de la présence d'un gaz d'intérêt étant effectuée à partir d'une valeur du paramètre représentatif du débit massique le long du chemin d'aspiration mesurée simultanément à la mesure de pression mesurant la valeur de pression correspondant à la valeur de référence de pression.

**31.** Procédé selon la revendication 30 considérée comme dépendante de la revendication 23, **caractérisé en ce que** la quantification de la présence du gaz d'intérêt comprend un calcul d'une proportion ou d'un volume du gaz d'intérêt selon la formule :

$$A^* \sqrt[2]{D_m} + B$$

Avec $D_m$ le paramètre représentatif du débit massique, et $A^*$ et $B$ des coefficients numériques de calibration.

**32.** Procédé selon la revendication 30 considérée comme dépendante de la revendication 24, **caractérisé en ce que** la quantification de la présence du gaz d'intérêt comprend un calcul d'une proportion ou d'un volume du gaz d'intérêt selon la formule :

$$M^* D_m + N$$

Avec $D_m$ le paramètre représentatif du débit massique, et $M^*$ et $N$ des coefficients numériques de calibration.

**33.** Procédé selon l'une quelconque des revendications 18 à 32, **caractérisé en ce que** le gaz d'intérêt comprend :

- de 0 à 100% d'une première molécule d'intérêt, et
- de 0 à 100% d'au moins une autre molécule d'intérêt,

le procédé selon l'invention comprenant en outre :

- une quantification de la présence des autres molécules d'intérêt au sein du gaz à analyser au moyen d'au moins un capteur (20) de gaz situé le long du chemin d'aspiration, et
- une quantification de la présence de la première molécule d'intérêt dans le gaz à analyser à partir de la quantification de la présence du gaz d'intérêt et de la quantification de la présence des autres molécules d'intérêt.

**34.** Procédé selon l'une quelconque des revendications 18 à 33, **caractérisé en ce que** l'au moins un chemin d'écoulement comprend un chemin de dilution se terminant par l'orifice, et **en ce qu'**il comprend, avant l'aspiration du gaz à analyser, une expiration d'un gaz de dilution s'écoulant le long du chemin de dilution jusque dans l'échantillon.

**Patentansprüche**

**1.** Vorrichtung (1) zum Testen einer Probe (13) mittels Gasstroms (25), enthaltend:

- eine Öffnung (2),
- Einrichtungen (3) zum Erzeugen eines Gasstroms (25) in der Vorrichtung entlang zumindest eines Strömungsweges, der durch die Öffnung führt,
- zumindest einen Drucksensor (5, 6), wobei jeder Drucksensor dazu ausgebildet ist, einen Druck des Gasstroms entlang zumindest eines Strömungsweges zu messen, und
- einen Massendurchflussmesser (4), der dazu ausgebildet ist, einen für den Massendurchfluss des Gasstroms entlang eines jeden Strömungsweges repräsentativen Parameter zu messen, bei welcher Vorrichtung
- der zumindest eine Strömungsweg einen an der Öffnung beginnenden Ansaugweg umfasst,
- die Einrichtungen zum Erzeugen des Gasstroms dazu ausgebildet sind, ein zu analysierendes Gas anzusaugen, so dass das zu analysierende Gas entlang des Ansaugweges strömt,
- der Ansaugweg sich innerhalb der Vorrichtung in Höhe einer Messbohrung (14) bereichsweise verjüngt,
- der zumindest eine Drucksensor einen Ansaugdrucksensor (6) enthält, der dazu ausgebildet ist, einen Druck des zu analysierenden Gases entlang des Ansaugweges zu messen,
- der Massendurchflussmesser dazu ausgebildet ist, einen für den Massendurchfluss des zu analysierenden Gases entlang des Ansaugweges repräsentativen Parameter zu messen, und
- die Vorrichtung ferner Recheneinrichtungen (7) enthält, die dazu ausgebildet sind, das Vorhandensein von interessierendem Gas in dem zu analysierenden Gas ausgehend von einer Messung von dem für den Massendurchfluss des zu analysierenden Gases entlang des Ansaugweges repräsentativen Parameter zu quantifizieren,

und **dadurch gekennzeichnet ist, dass** die Recheneinrichtungen dazu ausgebildet sind, das Vorhandensein von interessierendem Gas in Form von einer Berechnung eines Anteils bzw. eines Volumens des interessierenden Gases zu quantifizieren, der bzw. das von dem Durchmesser der Messbohrung abhängt.

2. Vorrichtung nach Anspruch 1, **dadurch gekennzeichnet, dass** der Massendurchflussmesser ein Massendurchflussmesser ist, der auf Basis der Wärmeleitung arbeitet.

3. Vorrichtung nach Anspruch 1 oder 2, **dadurch gekennzeichnet, dass** die Recheneinrichtungen dazu ausgebildet sind, das Vorhandensein von interessierendem Gas in Form von einer Berechnung eines Anteils an interessierendem Gas zu quantifizieren, und dass dieser Anteil ein Prozentanteil an interessierendem Gas in dem zu analysierenden Gas oder ein Anteil in Mol pro Liter zu analysierendem Gas ist.

4. Vorrichtung nach einem der vorangehenden Ansprüche, **dadurch gekennzeichnet, dass** der Ansaugdrucksensor entlang des Ansaugweges zwischen der Öffnung und der Messbohrung liegt.

5. Vorrichtung nach einem der vorangehenden Ansprüche, **dadurch gekennzeichnet, dass** der Massendurchflussmesser entlang des Ansaugweges liegt, so dass die Messbohrung entlang des Ansaugweges zwischen der Öffnung und dem Massendurchflussmesser liegt.

6. Vorrichtung nach einem der vorangehenden Ansprüche, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas zu quantifizieren, der bzw. das in linearer Weise von der Quadratwurzel des für den Massendurchfluss entlang des Ansaugweges repräsentativen Parameter abhängt.

7. Vorrichtung nach einem der Ansprüche 1 bis 5, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas zu quantifizieren, der bzw. das in linearer Weise von dem für den Massendurchfluss entlang des Ansaugweges repräsentativen Parameter abhängt.

8. Vorrichtung nach einem der vorangehenden Ansprüche, **dadurch gekennzeichnet, dass** die Recheneinrichtungen dazu ausgebildet sind, das Vorhandensein von interessierendem Gas ferner ausgehend von einer Druckmessung entlang des Ansaugweges durch den Ansaugdrucksensor zu quantifizieren.

9. Vorrichtung nach Anspruch 8 in Abhängigkeit von Anspruch 6, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas zu quantifizieren, der bzw. das in linearer Weise von dem Kehrwert der vierten Wurzel der Druckmessung entlang des Ansaugweges durch den Ansaugdrucksensor abhängt.

10. Vorrichtung nach Anspruch 9, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas nach der Formel

$$A\,\frac{\sqrt[2]{D_m}}{\sqrt[4]{P_r}} + B$$

zu quantifizieren, worin $D_m$ der für den Massendurchfluss repräsentative Parameter, $P_r$ der von dem Ansaugdrucksensor gemessene Druck, und A und B numerische Kalibrierungskoeffizienten sind.

11. Vorrichtung nach Anspruch 8 in Abhängigkeit von Anspruch 7, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas zu quantifizieren, der bzw. das in linearer Weise von dem Kehrwert der Druckmessung entlang des Ansaugweges durch den Ansaugdrucksensor abhängt.

12. Vorrichtung nach Anspruch 11, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas nach der Formel

$$M\,\frac{D_m}{P_r} + N$$

zu quantifizieren, worin $D_m$ der für den Massendurchfluss repräsentative Parameter, $P_r$ der durch den Ansaugdrucksensor gemessene Druck, und M und N numerische Kalibrierungskoeffizienten sind.

13. Vorrichtung nach einem der Ansprüche 1 bis 7, **dadurch gekennzeichnet, dass** die Recheneinrichtungen dazu ausgebildet sind, eine Quantifizierung des Vorhandenseins von interessierendem Gas bei einem durch den Ansaugdrucksensor gemessenen Druckwert entlang des Ansaugweges auszulösen, der einem Ansaugdruckreferenzwert entspricht, wobei die Recheneinrichtungen dazu ausgebildet sind, das Vorhandensein von interessierendem Gas ausgehend von einem Wert des für den Massendurchfluss entlang des Ansaugweges repräsentativen Parameter zu quantifizieren, der gleichzeitig mit der Druckmessung gemessen wird, welche den dem Ansaugdruckreferenzwert entsprechenden Druckwert misst.

14. Vorrichtung nach Anspruch 13 in Abhängigkeit von Anspruch 6, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas nach der Formel

$$A^* \sqrt[2]{D_m} + B$$

zu quantifizieren,
worin $D_m$ der für den Massendurchfluss repräsentative Parameter, und $A^*$ und $B$ numerische Kalibrierungskoeffizienten sind.

15. Vorrichtung nach Anspruch 13 in Abhängigkeit von Anspruch 7, **dadurch gekennzeichnet, dass** die Recheneinrichtungen dazu ausgebildet sind, das Vorhandensein von interessierendem Gas in Form von einer Berechnung eines Anteils bzw. eines Volumens an interessierendem Gas nach der Formel

$$M^* D_m + N$$

zu quantifizieren, worin $D_m$ der für den Massendurchfluss repräsentative Parameter, und $M^*$ und $N$ numerische Kalibrierungskoeffizienten sind.

16. Vorrichtung nach einem der vorangehenden Ansprüche, **dadurch gekennzeichnet, dass** die Recheneinrichtungen dazu ausgebildet sind, das Vorhandensein von einem ersten interessierenden Molekül des interessierenden Gases zu quantifizieren, wobei die Vorrichtung ferner entlang des Ansaugweges zumindest einen Gassensor (20) enthält, der dazu ausgebildet ist, das Vorhandensein von zumindest einem weiteren interessierenden Molekül des interessierenden Gases zu quantifizieren, wobei die Recheneinrichtungen dazu ausgebildet sind, das Vorhandensein von dem ersten interessierenden Molekül ausgehend von einer Quantifizierung des Vorhandenseins von dem interessierenden Gas und einer Quantifizierung des Vorhandenseins von weiteren interessierenden Molekülen zu quantifizieren.

17. Vorrichtung nach einem der vorangehenden Ansprüche, **dadurch gekennzeichnet, dass** der zumindest eine Strömungsweg einen Verdünnungsweg umfasst, der an der Öffnung endet, wobei die Einrichtungen zum Erzeugen des Gasstroms dazu ausgebildet sind, ein Verdünnungsgas entlang des Verdünnungsweges abzugeben.

18. Verfahren zum Testen einer Probe mittels Gasstroms (25), **dadurch gekennzeichnet, dass** es umfasst:

   - Ansaugen eines zu analysierenden Gases, das von einer Probe (13) stammt, wobei das zu analysierende, angesaugte Gas entlang eines Ansaugströmungsweges strömt, der an einer Öffnung (2) beginnt, die mit der Probe verbunden ist, wobei der Ansaugweg sich in Höhe einer Messbohrung (14) bereichsweise verjüngt,
   - Messen von dem Druck des zu analysierenden Gases entlang des Ansaugweges,
   - Messen von einem für den Massendurchfluss des zu analysierenden Gases entlang des Ansaugströmungsweges repräsentativen Parameter, und
   - Quantifizieren des Vorhandenseins von einem interessierenden Gas in dem zu analysierenden Gas ausgehend von der Messung von dem für den Massendurchfluss entlang des Ansaugweges repräsentativen Parameter,

   **dadurch gekennzeichnet, dass** das Quantifizieren des Vorhandenseins von dem interessierenden Gas ein Berechnen eines Anteils bzw. Volumens an interessierendem Gas umfasst, der bzw. das von dem Durchmesser der Messbohrung abhängt.

19. Verfahren nach Anspruch 18, **dadurch gekennzeichnet, dass** das Messen von einem für den Massendurchfluss repräsentativen Parameter ein Messen durch einen Massendurchflussmesser (4) ist, der auf Basis der Wärmeleitung arbeitet.

20. Verfahren nach Anspruch 18 oder 19, **dadurch gekennzeichnet, dass** das Quantifizieren des Vorhandenseins von interessierendem Gas ein Berechnen eines Anteils an interessierendem Gas umfasst und dass dieser Anteil ein Prozentanteil an interessierendem Gas in dem zu analysierenden Gas oder ein Anteil in Mol pro Liter an zu analysierendem Gas ist.

21. Verfahren nach einem der Ansprüche 18 bis 20, **dadurch gekennzeichnet, dass** das Messen des Drucks durch einen Ansaugdrucksensor (6) erfolgt, der entlang des Ansaugweges zwischen der Probe und der Messbohrung liegt.

22. Verfahren nach einem der Ansprüche 18 bis 21, **dadurch gekennzeichnet, dass** das Messen des für den Massendurchfluss repräsentativen Parameters durch einen Massendurchflussmesser erfolgt, der entlang des Ansaugweges liegt, so dass die Messbohrung entlang des Ansaugweges zwischen der Probe und dem Massendurchflussmesser liegt.

23. Verfahren nach einem der Ansprüche 18 bis 22, **dadurch gekennzeichnet, dass** das Quantifizieren des Vorhandenseins von interessierendem Gas ein Berechnen eines Anteils bzw. Volumens an interessierendem Gas umfasst, der bzw. das in linearer Weise von der Quadratwurzel des für den Massendurchfluss entlang des Ansaugweges repräsentativen Parameters abhängt.

24. Verfahren nach einem der Ansprüche 18 bis 22, **dadurch gekennzeichnet, dass** das Quantifizieren des Vorhandenseins von interessierendem Gas ein Berechnen eines Anteils bzw. eines Volumens an interessierendem Gas umfasst, der bzw. das in linearer Weise von dem für den Massendurchfluss entlang des Ansaugweges repräsentativen Parameter abhängt.

25. Verfahren nach einem der Ansprüche 18 bis 24, **dadurch gekennzeichnet, dass** das Quantifizieren des Vorhandenseins von interessierendem Gas ferner ausgehend von dem entlang des Ansaugweges gemessenen Druck erfolgt.

26. Verfahren nach Anspruch 25 in Abhängigkeit von Anspruch 23, **dadurch gekennzeichnet, dass** das Quantifizieren des Vorhandenseins von interessierendem Gas ein Berechnen eines Anteils bzw. eines Volumens an interessierendem Gas umfasst, der bzw. das in linearer Weise von dem Kehrwert der vierten Wurzel der Druckmessung entlang des Ansaugweges abhängt.

27. Vorrichtung nach Anspruch 26, **dadurch gekennzeichnet, dass** das Quantifizieren des Vorhandenseins von interessierendem Gas ein Berechnen eines Anteils bzw. eines Volumens an interessierendem Gas nach der Formel

$$A\, \frac{\sqrt[2]{D_m}}{\sqrt[4]{P_r}} + B$$

umfasst, worin $D_m$ der für den Massendurchfluss repräsentative Parameter, $P_r$ der gemessene Druck, und A und B numerische Kalibrierungskoeffizienten sind.

28. Verfahren nach Anspruch 25 in Abhängigkeit von Anspruch 24, **dadurch gekennzeichnet, dass** das Quantifizieren des Vorhandenseins von interessierendem Gas ein Berechnen eines Anteils bzw. eines Volumens an interessierendem Gas umfasst, der bzw. das in linearer Weise von dem Kehrwert von

$P_r$ des entlang des Ansaugweges gemessenen Drucks abhängt.

29. Verfahren nach Anspruch 28, **dadurch gekennzeichnet, dass** das Quantifizieren des Vorhandenseins von interessierendem Gas ein Berechnen eines Anteils bzw. eines Volumens an interessierendem Gas nach der Formel

$$M\, \frac{D_m}{P_r} + N$$

umfasst, worin $D_m$ der für den Massendurchfluss repräsentative Parameter, $P_r$ der durch den Ansaugdrucksensor gemessene Druck, und M und N numerische Kalibrierungskoeffizienten sind.

30. Verfahren nach einem der Ansprüche 18 bis 24, **dadurch gekennzeichnet, dass** das Quantifizieren des Vorhandenseins von interessierendem Gas bei einem gemessenen Druckwert entlang des Ansaugweges ausgelöst wird, der einem Ansaugdruckreferenzwert entspricht, wobei das Quantifizieren des Vorhandenseins von interessierendem Gas ausgehend von einem Wert des für den Massendurchfluss entlang des Ansaugweges repräsentativen Parameters erfolgt, der gleichzeitig mit der Druckmessung gemessen wird, welche den dem Ansaugdruckreferenzwert entsprechenden Druckwert misst.

31. Verfahren nach Anspruch 30 in Abhängigkeit von Anspruch 23, **dadurch gekennzeichnet, dass** das Quantifizieren des Vorhandenseins von interessierendem Gas ein Berechnen eines Anteils bzw. eines Volumens an interessierendem Gas nach der Formel

$$A^*\, \sqrt[2]{D_m} + B$$

umfasst,
worin $D_m$ der für den Massendurchfluss repräsentative Parameter, und A\* und B numerische Kalibrierungskoeffizienten sind.

32. Verfahren nach Anspruch 30 in Abhängigkeit von Anspruch 24, **dadurch gekennzeichnet, dass** das Quantifizieren des Vorhandenseins von interessierendem Gas ein Berechnen eines Anteils bzw. eines Volumens an interessierendem Gas nach der Formel

$$M^*\, D_m + N$$

umfasst, worin $D_m$ der für den Massendurchfluss repräsentative Parameter, und M\* und N numerische Kalibrierungskoeffizienten sind.

33. Verfahren nach einem der Ansprüche 18 bis 32, **dadurch gekennzeichnet, dass** das interessierende Gas enthält:

- 0 bis 100 % eines ersten interessierenden Moleküls, und
- 0 bis 100 % zumindest eines weiteren, interessierenden Moleküls, wobei das erfindungsgemäße Verfahren ferner umfasst:
- Quantifizieren des Vorhandseins weiterer interessierender Moleküle in dem zu analysierenden Gas mittels zumindest eines Gassensors (20), der entlang des Ansaugweges liegt, und
- Quantifizieren des Vorhandenseins von zumindest dem ersten interessierenden Molekül in dem zu analysierenden Gas ausgehend von dem Quantifizieren des Vorhandenseins von interessierendem Gas und von dem Quantifizieren des Vorhandenseins von weiteren interessierenden Molekülen.

34. Verfahren nach einem der Ansprüche 18 bis 33, **dadurch gekennzeichnet, dass** der zumindest eine Strömungsweg einen Verdünnungsweg umfasst, der an der Öffnung endet, und dass es vor dem Ansaugen des zu analysierenden Gases ein Abgeben eines Verdünnungsgases entlang des Verdünnungsweges bis zur Probe umfasst.

## Claims

1. Device (1) for testing a sample (13) via a gas stream (25), comprising:

- an opening (2),
- means (3) for generating a gas stream (25) in the device along at least one flow path passing through the opening,
- at least one pressure sensor (5, 6), each pressure sensor being arranged in order to measure a pressure of the gas stream along at least one flow path, and
- a mass flowmeter (4), arranged in order to measure a parameter representing the mass flow rate of the gas stream along each flow path,

**characterized in that**:

- the at least one flow path comprises a suction path starting at the opening,
- the means for generating the gas stream are arranged in order to suck in a gas to be analyzed so that this gas to be analyzed flows along the suction path,
- within the device, said suction path narrows locally at a measurement hole (14),
- the at least one pressure sensor comprises a suction pressure sensor (6) arranged in order to measure a pressure of the gas to be analyzed along the suction path,
- the mass flowmeter is arranged in order to measure a parameter representing the mass flow rate of the gas to be analyzed along the suction path, and
- the device also comprises calculation means (7) arranged in order to quantify the presence of a gas of interest within the gas to be analyzed, based on a measurement of the parameter representing the mass flow rate of the gas to be analyzed along the suction path, and **characterized in that** the calculation means are arranged in order to quantify the presence of the gas of interest in the form of a calculation of a _proportion or of a volume of the gas of interest which depends on the diameter of the measurement hole.

2. Device according to claim 1, **characterized in that** the mass flowmeter is a mass flowmeter using thermal conductivity.

3. Device according to claim 1 or 2, **characterized in that** the calculation means are arranged in order to quantify the presence of the gas of interest in the form of a calculation of a proportion of the gas of interest, and **in that** this proportion is a proportion in percentage of the gas of interest in the gas to be analyzed or a proportion in mol per litre of gas to be analyzed.

4. Device according to any one of the preceding claims, **characterized in that** the suction pressure sensor is situated along the suction path between the opening and the measurement hole.

5. Device according to any one of the preceding claims, **characterized in that** the mass flowmeter is situated along the suction path so that the measurement hole is situated along the suction path between the opening and the mass flowmeter.

6. Device according to any one of the preceding claims, **characterized in that** the calculation means are arranged in order to quantify the presence of the gas of interest in the form of a calculation of a proportion or of a volume of the gas of interest which depends affinely on the square root of the parameter representing the mass flow rate along the suction path.

7. Device according to any one of claims 1 to 5, **characterized in that** the calculation means are arranged in order to quantify the presence of the gas of interest in the form of a calculation of a proportion or of a volume of the gas of interest which depends affinely on the parameter representing the mass flow rate along the suction path.

8. Device according to any one of the preceding claims, **characterized in that** the calculation means are arranged in order to quantify the presence of the gas of interest also based on a measurement of pressure along the suction path by the suction pressure sensor.

9. Device according to claim 8 considered as dependent on claim 6, **characterized in that** the calculation means are arranged in order to quantify the presence of the gas of interest in the form of a calculation of a proportion or of a volume of the gas of interest which depends affinely on the inverse fourth root of the measurement of pressure along the suction path by the suction pressure sensor.

10. Device according to claim 9, **characterized in that** the calculation means are arranged in order to quantify the presence of the gas of interest in the form of a calculation of a proportion or of a volume of the gas of interest according to the formula:

$$A\frac{\sqrt[2]{D_m}}{\sqrt[4]{P_r}} + B$$

With $D_m$ the parameter representing the mass flow rate, $P_r$ the pressure measured by the suction pressure sensor, and A and B being numerical calibration coefficients.

11. Device according to claim 8 considered as dependent on claim 7, **characterized in that** the calculation means are arranged in order to quantify the presence of the gas of interest in the form of a calculation of a proportion or of a volume of the gas of interest which depends affinely on the inverse of the measurement of pressure along the suction path by the suction pressure sensor.

12. Device according to claim 11, **characterized in that** the calculation means are arranged in order to quantify the presence of the gas of interest in the form of a calculation of a proportion or of a volume of the gas of interest according to the formula:

$$M\frac{D_m}{P_r} + N$$

With $D_m$ the parameter representing the mass flow rate, $P_r$ the pressure measured by the suction pressure sensor, and M and N being numerical calibration coefficients.

13. Device according to any one of claims 1 to 7, **characterized in that** the calculation means are arranged in order to trigger a quantification of the presence of the gas of interest for a value of the pressure along the suction path measured by the suction pressure sensor corresponding to a suction pressure reference value, the calculation means being arranged in order to quantify the presence of the gas of interest based on a value of the parameter representing the mass flow rate along the suction path measured simultaneously with the pressure measurement measuring the pressure value corresponding to the suction pressure reference value.

14. Device according to claim 13 considered as dependent on claim 6, **characterized in that** the calculation means are arranged in order to quantify the presence of the gas of interest in the form of a calculation of a proportion or of a volume of the gas of interest according to the formula:

$$A^*\sqrt[2]{D_m} + B$$

With $D_m$ the parameter representing the mass flow rate, and A* and B being numerical calibration coefficients.

15. Device according to claim 13 considered as dependent on claim 7, **characterized in that** the calculation means are arranged in order to quantify the presence of the gas of interest in the form of a calculation of a proportion or of a volume of the gas of interest according to the formula:

$$M^*D_m + N$$

With $D_m$ the parameter representing the mass flow rate, and M* and N being numerical calibration coefficients.

16. Device according to any one of the preceding claims, **characterized in that** the calculation means are moreover arranged in order to quantify the presence of a first molecule of interest of the gas of interest, the device also comprising along the suction path at least one gas sensor (20) arranged in order to quantify the presence of at least one other molecule of interest of the gas of interest, the calculation means being arranged in order to quantify the presence of the first molecule of interest based on a quantification of the presence of the gas of interest and a quantification of the presence of the other molecules of interest.

**17.** Device according to any one of the preceding claims, **characterized in that** the at least one flow path comprises a dilution path terminating at the opening, the means for generating the gas stream being arranged in order to exhaust a dilution gas along the dilution path.

**18.** process for testing a sample via a gas stream (25), **characterized in that** it comprises:

- sucking a gas to be analyzed originating from a sample (13), said sucked gas to be analyzed flowing along a suction path 40 starting at an opening (2) linked to the sample, said suction path narrowing locally at a measurement hole (14),
- a pressure measurement of the gas to be analyzed along the suction path,
- a measurement of a parameter representing the mass flow rate of the gas to be analyzed along the suction path, and
- a quantification of the presence of a gas of interest within the gas to be analyzed, based on the measurement of the parameter representing the mass flow rate along the suction path,

**characterized in that** the quantification of the presence of the gas of interest comprises a calculation of a proportion or of a volume of the gas of interest which depends on the diameter of the measurement hole.

**19.** Process according to claim 18, **characterized in that** the measurement of a parameter representing the mass flow rate is a measurement by a mass flowmeter (4) using thermal conductivity.

**20.** Process according to claim 18 or 19, **characterized in that** the quantification of the presence of the gas of interest comprises a calculation of a proportion of the gas of interest, and **in that** this proportion is a proportion in percentage of gas of interest in the gas to be analyzed or in mol per litre of gas to be analyzed.

**21.** Process according to any one of claims 18 to 20, **characterized in that** the pressure measurement is carried out by a suction pressure sensor (6) situated along the suction path between the sample and the measurement hole.

**22.** Process according to any one of claims 18 to 21, **characterized in that** the measurement of the parameter representing the mass flow rate is carried out by a mass flowmeter situated along the suction path so that the measurement hole is situated along the suction path between the sample and the mass flowmeter.

**23.** Process according to any one of claims 18 to 22, **characterized in that** the quantification of the presence of the gas of interest comprises a calculation of a proportion or of a volume of the gas of interest which depends affinely on the square root of the parameter representing the mass flow rate along the suction path.

**24.** Process according to any one of claims 18 to 22, **characterized in that** the quantification of the presence of the gas of interest comprises a calculation of a proportion or of a volume of the gas of interest which depends affinely on the parameter representing the mass flow rate along the suction path.

**25.** Process according to any one of claims 18 to 24, **characterized in that** the quantification of the presence of a gas of interest is carried out also based on the pressure measured along the suction path.

**26.** Process according to claim 25 considered as dependent on claim 23, **characterized in that** the quantification of the presence of the gas of interest comprises a calculation of a proportion or of a volume of the gas of interest which depends affinely on the inverse of the fourth root of the measurement of pressure along the suction path.

**27.** Process according to claim 26, **characterized in that** the quantification of the presence of the gas of interest comprises a calculation of a proportion or of a volume of the gas of interest according to the formula:

$$A \frac{\sqrt[2]{D_m}}{\sqrt[4]{P_r}} + B$$

With $D_m$ the parameter representing the mass flow rate, $P_r$ the pressure measured, and A and B being numerical calibration coefficients.

**28.** Process according to claim 25 considered as dependent on claim 24, **characterized in that** the quantification of the presence of the gas of interest comprises a calculation of a proportion or of a volume of the gas of interest which depends affinely on the inverse of $P_r$, the pressure measured along the suction path.

**29.** Process according to claim 28, **characterized in that** the quantification of the presence of the gas of interest comprises a calculation of a proportion or of a volume of the gas of interest according to the formula:

$$M \frac{D_m}{P_r} + N$$

With $D_m$ the parameter representing the mass flow rate, $P_r$ the pressure measured, M and N being numerical calibration coefficients.

**30.** Process according to any one of claims 18 to 24, **characterized in that** the quantification of the presence of a gas of interest is triggered in the case of a value of the pressure measured along the suction path corresponding to a suction pressure reference value, the quantification of the presence of a gas of interest being carried out based on a value of the parameter representing the mass flow rate along the suction path measured simultaneously with the pressure measurement measuring the pressure value corresponding to the pressure reference value.

**31.** Process according to claim 30 considered as dependent on claim 23, **characterized in that** the quantification of the presence of the gas of interest comprises a calculation of a proportion or of a volume of the gas of interest according to the formula:

$$A^* \sqrt[2]{D_m} + B$$

With $D_m$ the parameter representing the mass flow rate, and A* and B being numerical calibration coefficients.

**32.** Process according to claim 30 considered as dependent on claim 24, **characterized in that** the quantification of the presence of the gas of interest comprises a calculation of a proportion or of a volume of the gas of interest according to the formula:

$$M^* D_m + N$$

With $D_m$ the parameter representing the mass flow rate, and M* and N being numerical calibration coefficients.

**33.** Process according to any one of claims 18 to 32, **characterized in that** the gas of interest comprises:

- from 0 to 100% of a first molecule of interest, and
- from 0 to 100% of at least one other molecule of interest,

the process according to the invention also comprising:

- a quantification of the presence of the other molecules of interest within the gas to be analyzed by means of at least one gas sensor (20) situated along the suction path, and
- a quantification of the presence of the first molecule of interest in the gas to be analyzed based on the quantification of the presence of the gas of interest and the quantification of the presence of the other molecules of interest.

**34.** Process according to any one of claims 18 to 33, **characterized in that** the at least one flow path comprises a dilution path terminating at the opening, and **in that** it comprises, before sucking the gas to be analyzed, exhausting of a dilution gas flowing along the dilution path into the sample.

FIG. 1

FIG. 2

FIG. 3

FIG. 4

FIG. 5

FIG. 6

FIG. 7

FIG. 8

## RÉFÉRENCES CITÉES DANS LA DESCRIPTION

**Documents brevets cités dans la description**

- WO 2008046420 A1

- US 2011303019 A1

**Littérature non-brevet citée dans la description**

- **SEARS W. M. et al.** Surface adsorption and gas consumption in restricted flow, thermally driven, gas sensors. *semiconductor science and technology,* 01 Janvier 1990, vol. 5